(19) Europäisches Patentamt
European Patent Office
Office européen des brevets

(11) EP 1 409 500 B1

(12) EUROPEAN PATENT SPECIFICATION

(45) Date of publication and mention of the grant of the patent: **01.03.2006 Bulletin 2006/09**

(21) Application number: **01928114.6**

(22) Date of filing: **11.05.2001**

(51) Int Cl.:
*C07H 15/207* (2006.01) *C07H 11/04* (2006.01)
*C07H 5/06* (2006.01) *A61K 31/7034* (2006.01)
*A61P 5/50* (2006.01)

(86) International application number: **PCT/GB2001/002093**

(87) International publication number: **WO 2001/085747 (15.11.2001 Gazette 2001/46)**

(54) **ALKYLATED INOSITOLGLYCANS AND THEIR USES**

ALKYLIERTE INOSITOLGLYCANE UND IHRE VERWENDUNG

INOSITOLGLYCANES ALKYLES ET LEUR UTILISATION

(84) Designated Contracting States:
**AT BE CH CY DE DK ES FI FR GB GR IE IT LI LU MC NL PT SE TR**

(30) Priority: **12.05.2000 GB 0011591**
**12.05.2000 US 203607 P**
**02.03.2001 US 798125**

(43) Date of publication of application: **21.04.2004 Bulletin 2004/17**

(73) Proprietor: **Rodaris Pharmaceuticals Limited Oxford, OX4 4GA (GB)**

(72) Inventors:
- **MARTIN-LOMAS, Manuel Editor Jose Manuel Lara E-41013 Seville (ES)**
- **RADEMACHER, Thomas, William Oxfordshire OX1 5EY (GB)**
- **CARO, Hugo, Noberto Barcelona 08036 (ES)**
- **FRANCIOS, Irene Woking Surrey GU21 4BE (GB)**

(74) Representative: **Kiddle, Simon John et al Mewburn Ellis LLP York House, 23 Kingsway London WC2B 6HP (GB)**

(56) References cited:
**US-A- 5 550 166** **US-A- 5 652 221**

- **C. DERAPPE ET AL.: "Characterization of a new oligosaccharide containing myo-inositol found in pregnancy urine" CARBOHYDRATE RESEARCH, vol. 115, 1983, pages 221-229, XP002171185**
- **S.J. ANGYAL, B. SHELTON: "Cyclitols. Part XXII. Synthesis of some mannosyl- and mannosyl-mannosyl-myoinositols, and of galactinol" J. CHEM. SOC. (C), 1966, pages 433-438, XP002171191**
- **FRICK W ET AL: "STRUCTURE - ACTIVITY RELATIONSHIP OF SYNTHETIC PHOSPHOINOSITOLGLYCANS MIMICKING METABOLIC INSULIN ACTION" BIOCHEMISTRY, AMERICAN CHEMICAL SOCIETY. EASTON, PA, US, vol. 37, no. 38, 1998, pages 13421-13436, XP000946196 ISSN: 0006-2960**

EP 1 409 500 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

### Field of the Invention

The present invention relates to compounds and their uses, and in particular to compounds which have a mimetic or antagonistic property of an inositol phosphoglycan or a free GPI precursor of an IPG, and the uses of these compounds, e.g. to treat a condition ameliorated by administration of an IPG second messenger or an IPG antagonist thereof.

### Background of the Invention

Many of the actions of growth factors and hormones on cells are thought to be mediated by a family of inositol phosphoglycan (IPG) second messengers[13]. It is thought that the source of IPGs is a "free" form of glycosyl phosphatidylinositol (GPI) situated in cell membranes. IPGs are thought to be released by the action of phosphatidylinositol-specific phospholipases following binding of growth factors to receptors on the cell surface. There is evidence that IPGs mediate the action of a large number of growth factors including insulin, nerve growth factor, hepatocyte growth factor, insulin-like growth factor I (IGF-I), fibroblast growth factor, transforming growth factor β, the action of IL-2 on B-cells and T-cells, ACTH signalling of adrenocortical cells, IgE, FSH and hCG stimulation of granulosa cells, thyrotropin stimulation of thyroid cells, cell proliferation in the early developing ear and rat mammary gland.

Partially characterised inositolphosphoglycans (IPGs) have been postulated to mediate the action of a number of growth factors and hormones including insulin and insulin-like growth factor I (IGF-I)[1]. Despite their isolation from several tissues type, the precise chemical structures of these IPGs are, however, still unknown and two main structural groups have been proposed on the basis of the chemical coinposition[2,3] which display different biological activity and tissue distribution[4]; the family of glucosamine-*myo*-inositol containing IPGs (IPG-A) and the family of *chiro*-inositol-galactosamine containing IPGs (IPG-P).

In an attempt to establish the minimal structural requirements for biological activity, a number of compounds containing some of the basic structural motifs that have been postulated for IPG mediators have been synthesised in the art[5]. These synthetic compounds include *O*-(2-amino-2-deoxy-D-glucopyranosyl)-α(1-6)-*chiro*-inoaitol 1-phosphate and *O*-(2-amino-2-deoxy-D-glucopyranosyl)-α(1-6)-*myo*-inositol 1-phosphate[6].

US Patent No: 6,004,938 (Hoechst) and Frick et al (Biochemistry, 37138):13421-36, 1998) discloses a group of synthetic inositol glycans having insulin-like action. The compounds are based on 2-6 monsaccharide units linked to an inositol moiety. The examples in the patent all employ myo-inositol and are composed of 5 or 6 units apart from two pseudo-trisaccharide compounds G and H. Compounds G and H are HO-PO(H)O-6Man-α(1-4)-GluN-α(1-6)-(L)inositol-1,2(cyclic) phosphate and HO-PO(H)O-6Man-α(1-4)-GluN-α(1-6)-(L)inositol, otherwise known as *O*-(6-hydrogenphosphonate-α-D-mannopyranosyl)-(1-4)-(2-ammonio-2-deoxy-α-D-glucopyranosyl)-(1-6)-L-*myo*-inositol-1,2-cyclic phosphate and *O*-(6-hydrogenphosphonate-α-D-mannopyranosyl)-(1-4)-(2-amino-2-deoxy-α-D-glucopyranosyl)-L-*myo*-inositol. The properties of exemplified compounds are investigated in lipogenesis and glucose transport assays employing rat fat cells.

WO96/14075 or US 5652221 (University of Virginia) discloses a generic family of compounds D-hexosamines linked to an inositol via a β1,4-linkage. The inositols can be *myo* or *chiro*-inositol or pinitol, while the hexosamines are glucosamine or galactosamine. However, this application describes the synthesis of just two compounds 4-*O*-(2-deoxy-2-amino-β-D-galactopyranosyl)-D-pinitol and 4-*O*-(2-deoxy-2-amino-β-D-galactopyranosyl)-D-*chiro*-inositol, or in IUPAC notation *O*-(2-amino-2-deoxy-β-D-galactopyranosyl)-(1-4)-D-pinitol and *O*-(2-amino-2-deoxy-β-D-galactopyranosyl)-(1-4)-D-chiro-inositol.

Angyd and Carter (J. Chem. Soc. Org., 1966(4), 433-438) discloses two α- β- mannopyranosyl-myo inositols.

WO99/06421 (University of Virginia) describes synthetic insulin mimetic substances and includes a general formula I showing β1,4-linked disaccharides. However, despite this the compounds synthesised in this application are exactly the same as those disclosed in the applicant's earlier application, WO96/14075.

A multi-step synthesis of a IPG-P mimetic from glucose has been previously reported in Jaramillo et al[6], which discloses a compound called C4, 1D-6-*O*-(2-amino-2-deoxy-α-D-glucopyranosyl)-*chiro*-inositol-1-phosphate. A further synthesis of this compound is described in our co-pending International Patent Application PCT/GB99/03715 (Rademacher Group Limited).

Derappe et al (Carbohydrate Res., 115:221-229, 1980), discloses the characterisation of an oligosaccharide containing myo-inositol isolated from pregnancy urine.

It remains a significant problem in the art to produce synthetic compounds which can mimic one or more of the activities of inositol phosphoglycans or which act as antagonists of IPGs.

## Summary of the Invention

Broadly, the present invention relates to IPG mimetic and antagonist compounds, and in particular to compounds based on the *chiro*-inositol derivative pinitol (3-*O*-methyl-*chiro*-inositol), It also relates to methods of producing the compounds and to their medical uses. The compounds disclosed herein are useful as synthetic mimetics of IPG-P or IPG-A second messengers and/or growth factors or hormones whose action is mediated by IPGs, as synthetic mimetics of free GPI precursors of IPGs, or as competitive antagonists of IPGs.

Accordingly, in a first aspect, the present invention provides a compound represented by the general formula:

**X- 1,6-pinitol**

wherein:

X represents a sugar residue;
the sugar residue is substituted with between one, two or three groups, and the pinitol is unsubstituted or is further substituted with between one and four groups, the group or groups being independently selected from:

(a) a phosphoryl group which is phosphate -O-P(O)$(OH)_2$; thiophosphate -O-P(S)$(OH)_2$; a phosphate ester -O-P (O)$(OR)_2$; a thiophosphate ester -O-P(S)$(OR)_2$; phosphonate - O-P(O)OHR; thiophosphonate -O-P(S)OHR; substituted phosphonate -O-P(O)$OR_1R_2$; substituted thiophosphonate -O-P(S)$OR_1R_2$; -O-P(S)(OH)(SH); or cyclic phosphate - O-P(O)$(OR)_2$;
(b) a phosphorus containing group which is phosphoramidite -O-P(OR)-$NR_1R_2$ or phosphoramidate -O-P(O) (O*)-$NR_1R_2$;
(c) a sulphur group which is -O-S(O)$(OH)_2$, -SH, -SR, -S(→O)-R, -S$(O)_2$R, RO-S$(O)_2^-$, -O-$SO_2NH_2$, -O-$SO_2R_1R_2$ or sulphamide -$NHSO_2NH_2$;
(d) an amino group which is -NHR, -$NR_1R_2$, -NHAc, NHCOR, -NH-O-COR, -$NHSO_3^-$, or -$NHSO_2R$, -N($SO_2$R), or an amidino group which is -NH-C(=NH)$NH_2$ or an ureido group which is -NH-CO-$NR_1R_2$ or a thiouriedo group which is -NH-C(S)-$NH_2$;
(e) a hydroxy group or a substituted hydroxy groups represented by -$OR_3$, where $R_3$ is $C_{1-10}$ unsubstituted or substituted alkyl, $CHF_2$, $CF_3$, alkoxyalkyl, aryloxyalkyl, cycloalkyl, alkenyl (unsubstituted alkyl), alkylene ($C_{3-7}$ cycloalkyl), -OCOR, aryl, heteroaryl or acetal, or where two hydroxyl groups are joined as a ketal;
(f) a halogen substituent such as fluorine or chlorine;
(g) hydrogen to provide a deoxy sugar.

wherein R, $R_1$ and $R_2$ are independently hydrogen or $C_{1-10}$ unsubstituted or substituted alkyl or aryl;
or a derivative thereof.

The compounds may be provided as racemic or diasteromeric mixtures, resolved or partially resolved optical isomers, and as pharmaceutically acceptable salts, esters and derivatives as discussed in more detail below.

Preferably, the sugar residue is a hexose or a pentose, and may be an aldose or a ketose. The sugar residue can a member of the D or L series and can include amino sugars, deoxy sugars and their uronic acid derivatives. Preferably, where the sugar residue is a hexose, it is selected from the group consisting of glucose, galactose or mannose, or substituted hexose sugar residues such as an amino sugar residue such as hexosamine, galactosamine or glucosamine, and more preferably D-glucosamine (2-amino-2-deoxy-D-glucose) or D-galactosamine (2-amino-2-deoxy-D-galactose). Preferred pentose sugar residues include arabinose, fucose and ribose. The sugar residue is optionally substituted at one, two, three or four positions, other than anomeric position or the position of linkage to the cyclitol moiety.

The cyclitol can be the D or L enantiomer. The alkyl group at the 3-position of the cyclitol is preferably $C_{1-10}$ alkyl, and may be a substituted or unsubstituted primary, secondary or tertiaty alkyl group. Examples of substituted groups include $CF_3$, X$(CH_2)_n$-O- (where X is hydrogen, or substituted or unsubstituted alkyl), $CHF_2$O-. A preferred alkyl group is methyl when the cyclitol is D or L-pinitol (3-*O*-methyl-*chiro*-inositol), and is optionally substituted at one or more of the positions other than the 3-position or the position of linkage to the sugar residue. In further embodiments, the cyclitol may have one or more of the hydroxyl groups through which the substituents described above are removed so that any substituent(s) are linked to the ring carbon atom. The cyclitol moiety is optionally further substituted at one, two, three or four positions, other than the position of linkage to the sugar residue.

The linkage position of the sugar residue to the cyclitol is a 1,6 linkage. The linkage between the units is preferably via one of the oxygen atoms of the cyclitol moiety. However, this oxygen atom can be replaced one or more

times by $-CH_2-$ or -S- groups. Compounds with α or β linkages form part of the present invention.

To avoid confusion, the numbering system used herein is clarified with reference to the following structures. Importantly, as the *chiro*-inositol molecule contains a C2 plane of symmetry and positions 1 and 6 are equivalents. Therefore, for instance, compounds containing a β(1,6) linkage can be regarded as β(1,1) if there are no other substituents to define priority in the numbering system. Monosaccharide residues and oligosaccharides are named and numbered according to the recommendation proposed by the Joint Commission on Biochemical Nomenclature, International Union of Pure and Applied Chemistry and International Union of Biochemistry and Molecular Biology.

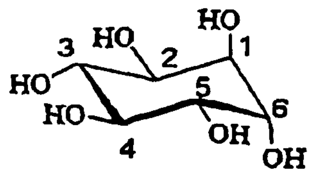

*chiro*-Inositol

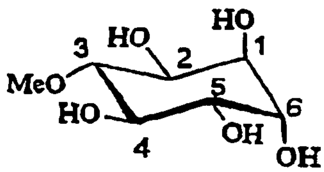

Pinitol: 3-*O*-Methyl-*chiro*-inositol

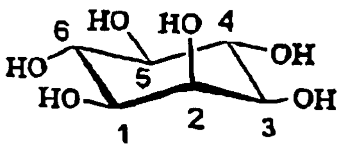

myo-Inositol

These compounds may be either α1,6 or β1,6 linked, and examples showing the synthesis of these compounds are provided in the description below. The synthesis of compounds 1-4, bearing the glucosaminyl α and β 1-6 (1, 2) and the galactosaminyl a and β 1-6 (3, 4) structural motifs from 3-*O*-methyl-D-*chiro*-inositol (D-pinitol) is reported below. Examples of α-linked compounds of the invention are compounds RGL1024 and 1025, the synthesis of which is described in detail below. Examples of β-linked compounds of the invention are compound RGL1015, the synthesis of which is described in detail below, and compound RGL1119.

In preferred embodiments, the present invention provides a compound selected from the group consisting of RGL1015, RGL1024, RGL1025 and RGL1119, and substituted forms and derivatives thereof as defined above.

| | |
|---|---|
| RGL1015 | *O*-(2-amino-2-deoxy-D-glucopyranosyl)-β(1,6)-D-3-*O*-methyl-*chiro*-inositol. |
| RGL1024 | *O*-(2-amino-2-deoxy-D-glucopyranosyl)-α(1,6)-D-3-*O*-methyl-*chiro*-inositol. |
| RGL1025 | *O*-(2-amino-2deoxy-D-galactopyranosyl)-α(1,6)-D-3-*O*-methyl-*chiro*-inositol. |
| RGL1119 | 1'-D-6-*O*-(T-amino-2-deoxy-β-D-galactopyranosyl)-3-*O*-methyl-D-*chiro*-inositol. |

In a further aspect, the present invention provides methods for making the compounds of the invention or their intermediates as set out in the following experimental description and the schemes. In a further related aspect, the present invention further relates to compounds which are the novel intermediates described herein. In particular, experiments with intermediates have shown that they can share biological activities with final or deprotected compounds and may therefore be useful therapeutics in their own right.

In a further aspect, the present invention provides one or more of the above compounds for use in a method of medical treatment. The compounds may be useful as IPG mimetics or IPG antagonists, e.g. as competitive antagonists.

In a further aspect, the present invention provides the use of one or more of the above compounds for the preparation of a medicament for the treatment of a condition ameliorated by the administration of an inositol phosphoglycan (IPG) second messenger or an IPG antagonist. Examples of such conditions are set out in the pharmaceutical uses section below.

Embodiments of the invention will now be described by way of example and not limitation with reference to the accompanying drawings.

## Brief Description of the Figures

Scheme 1 shows the synthesis of building block 7 by bis-protection of the trans-diequatorially oriented hydroxyl groups of D-pinitol 5 as cyclohexane 1,2-diacetal and the cis-oriented hydroxyl groups as isopropylidene acetal.
Scheme 2 shows the glycosylation of D-pinitol building block 7 with the 2-azido-2-deoxy-D-glucopyranosyl trichloroacetimidate 8 to give the IPG-like compounds 1 and 2.
Scheme 3 shows the glycosylation of D-pinitol building block 7 with the Z-azido-2-deoxy-D-galactopyranosyl trichloroscetimidate 13 to give the IP G-like compounds 3 and 4.
Scheme 4 shows the synthesis leading to compound RGL1119.
Figure 1 is a graph showing the PDH phosphates activation of exemplary compounds of the invention as compared to D-pinitol.

## Detailed Description

## Inositol phosphoglycans (IPGs)

IPG-A mediators modulate the activity of a number of insulin-dependent enzymes such as cAMP dependent protein kinase (inhibits), adenylate cyclase (inhibits) and cAMP phospho-diesterases (stimulates). In contrast, IPG-P mediators modulate the activity of insulin-dependent enzymes such as pyruvate dehydrogenase phosphatase (stimulates) and glycogen synthase phosphatase (stimulates). The A-type mediators mimic the lipogenic activity of insulin on adipocytes, whereas the P-type mediators mimic the glycogenic activity of insulin on muscle. Both A-and P-type mediators are mitogenic when added to fibroblasts in serum free media. The ability of the mediators to stimulate fibroblast proliferation is enhanced if the cells are transfected with the EGF-receptor. A-type mediators can stimulate cell proliferation in the chick cochleovestibular ganglia.
Soluble IPG fractions having A-type and P-type activity have been obtained from a variety of animal tissues including rat tissues (liver, kidney, muscle, brain, adipose, heart) and bovine liver. IPG-A and IPG-P biological activity has also been detected in human liver and placenta, malaria parasitized RBC and mycobacteria. The ability of an anti-inositolglycan antibody to inhibit insulin action on human placental cytotrophoblasts and BC3H1 myocytes or bovine-derived IPG action on rat diaphragm and chick cochleovestibular ganglia suggests cross-species conservation of many structural features. However, it is important to note that although the prior art includes these reports of IPG-A and IPG-P activity in some biological fractions, the purification or characterisation of the agents responsible for the activity is not disclosed.
IPG-A substances are cyclitol-containing carbohydrates, also containing $Zn^{2+}$ ions and phosphate and having the properties of regulating lipogenic activity and inhibiting cAMP dependent protein kinase. They may also inhibit adenylate cyclase, be mitogenic when added to EGF-transfected fibroblasts in serum free medium, and stimulate lipogenesis in adipocytes.
IPG-P substances are cyclitol-containing carbohydrates, also containing $Mn^{2+}$ and/or $Zn^{2+}$ ions and phosphate and having the properties of regulating glycogen metabolism and activating pyruvate dehydrogenase phosphatase. They may also stimulate the activity of glycogen synthase phosphatase, be mitogenic when added to fibroblasts in serum free medium, and stimulate pyruvate dehydrogenase phosphatase.
Methods for obtaining A-type and P-type mediators are set out in Caro et al, 1997, and in WO98/11116 and WO98/11117. Protocols for determining characteristic IPG biological activities such as PDH activation, PKA inhibition, acetylCoA activation, fructose-1,6-bis-phosphatase activity and lipogenesis are well known in the art, e.g. as described in Caro et al[14].

## Drug Formulation

The compounds of the invention may be derivatised in various ways. As used herein "derivatives" of the compounds includes salts, coordination complexes with metal ions such as $Mn^{2+}$ and $Zn^{2+}$, esters such as *in vivo* hydrolysable esters, free acids or bases, hydrates, prodrugs or lipids, coupling partners, and protecting groups.
Salts of the compounds of the invention are preferably physiologically well tolerated and non toxic. Many examples of salts are known to those skilled in the art. Compounds having acidic groups, such as phosphates or sulfates, can form salts with alkaline or alkaline earth metals such as Na, K, Mg and Ca, and with organic amines such as triethylamine and Tris (2-hydroxyethyl)amine. Salts can be formed between compounds with basic groups, e.g. amines, with inorganic acids such as hydrochloric acid, phosphoric acid or sulfuric acid, or organic acids such as acetic acid, citric acid, benzoic acid, fumaric acid, or tartaric acid. Compounds having both acidic and basic groups can form internal salts.
Esters can be formed between hydroxyl or carboxylic acid groups present in the compound and an appropriate

carboxylic acid or alcohol reaction partner, using techniques well known in the art.

Derivatives which as prodrugs of the compounds are convertible *in vivo* or *in vitro* into one of the parent compounds. Typically, at least one of the biological activities of compound will be reduced in the prodrug form of the compound, and can be activated by conversion of the prodrug to release the compound or a metabolite of it. An example of prodrugs are glycolipid derivatives in which one or more lipid moieties are provided as substituents on the sugar residue or the cyclitol moiety, leading to the release of the free form of the compound by cleavage with a phospholipase enzyme. Examples of prodrugs include the use of protecting groups which may be removed *in situ* releasing active compound or serve to inhibit clearance of the drug *in vivo.* Protecting groups are well known in the art and are discussed further below. An example of a suitable protecting group that might be used as a prodrug is the azido group used in the synthesis below, e.g. on the 2-position of the sugar moiety.

Other derivatives include coupling partners of the compounds in which the compounds is linked to a coupling partner, e.g. by being chemically coupled to the compound or physically associated with it. Examples of coupling partners include a label or reporter molecule, a supporting substrate, a carrier or transport molecule, an effector, a drug, an antibody or an inhibitor. Coupling partners can be covalently linked to compounds of the invention via an appropriate functional group on the compound such as a hydroxyl group, a carboxyl group or an amino group. Other derivatives include formulating the compounds with liposomes.

## Pharmaceutical Compositions

The compounds described herein or their derivatives can be formulated in pharmaceutical compositions, and administered to patients in a variety of forms, in particular to treat conditions which are ameliorated by the administration of inositol phosphoglycan second messengers or IPG antagonists such as competitive antagonist.

Pharmaceutical compositions for oral administration may be in tablet, capsule, powder or liquid form. A tablet may include a solid carrier such as gelatin or an adjuvant or an inert diluent. Liquid pharmaceutical compositions generally include a liquid carrier such as water, petroleum, animal or vegetable oils, mineral oil or synthetic oil. Physiological saline solution, or glycols such as ethylene glycol, propylene glycol or polyethylene glycol may be included. Such compositions and preparations generally contain at least 0.1wt% of the compound.

Parental administration includes administration by the following routes: intravenous, cutaneous or subcutaneous, nasal, intramuscular, intraocular, transepithelial, intraperitoneal and topical (including dermal, ocular, rectal, nasal, inhalation and aerosol), and rectal systemic routes. For intravenous, cutaneous or subcutaneous injection, or injection at the site of affliction, the active ingredient will be in the form of a parenterally acceptable aqueous solution which is pyrogen-free and has suitable pH, isotonicity and stability. Those of relevant skill in the art are well able to prepare suitable solutions using, for example, solutions of the compounds or a derivative thereof, e.g. in physiological saline, a dispersion prepared with glycerol, liquid polyethylene glycol or oils.

In addition to one or more of the compounds, optionally in combination with other active ingredient, the compositions can comprise one or more of a pharmaceutically acceptable excipient, carrier, buffer, stabiliser, isotonicizing agent, preservative or anti-oxidant or other materials well known to those skilled in the art. Such materials should be non-toxic and should not interfere with the efficacy of the active ingredient. The precise nature of the carrier or other material may depend on the route of administration, e.g. orally or parentally.

Liquid pharmaceutical compositions are typically formulated to have a pH between about 3.0 and 9.0, more preferably between about 4.5 and 8.5 and still more preferably between about 5.0 and 8.0. The pH of a composition can be maintained by the use of a buffer such as acetate, citrate, phosphate, succinate, Tris or histidine, typically employed in the range from about 1 mM to 50 mM. The pH of compositions can otherwise be adjusted by using physiologically acceptable acids or bases.

Preservatives are generally included in pharmaceutical compositions to retard microbial growth, extending the shelf life of the compositions and allowing multiple use packaging. Examples of preservatives include phenol, meta-cresol, benzyl alcohol, para-hydroxybenzoic acid and its esters, methyl paraben, propyl paraben, benzalconium chloride and benzethonium chloride. Preservatives are typically employed in the range of about 0.1 to 1.0 % (w/v).

Preferably, the pharmaceutically compositions are given to an individual in a "prophylactically effective amount" or a "therapeutically effective amount" (as the case may be, although prophylaxis may be considered therapy), this being sufficient to show benefit to the individual. Typically, this will be to cause a therapeutically useful activity providing benefit to the individual. The actual amount of the compounds administered, and rate and time-course of administration, will depend on the nature and severity of the condition being treated. Prescription of treatment, e.g. decisions on dosage etc, is within the responsibility of general practitioners and other medical doctors, and typically takes account of the disorder to be treated, the condition of the individual patient, the site of delivery, the method of administration and other factors known to practitioners. Examples of the techniques and protocols mentioned above can be found in Remington's Pharmaceutical Sciences, 16th edition, Osol, A. (ed), 1980. By way of example, and the compositions are preferably administered to patients in dosages of between about 0.01 and 100mg of active compound per kg of body weight, and

more preferably between about 0.5 and 10mg/kg of body weight.

The composition may further comprise one or more other pharmaceutically active agents, either further compounds of the invention, inositol phosphoglycans, growth factors such as insulin, NGF or other growth factors listed below, or other drugs, e.g. those in use for the treatment of diabetes or other conditions set out below.

## Medical Uses

As set out above, IPGs are second messengers for a range of different growth factors and hormones, including insulin, nerve growth factor, hepatocyte growth factor, cndothelial growth factor (EGF), insulin-like growth factor I (IGF-I), fibroblast growth factor, transforming growth factor β, the action of IL-2 on B-cells and T-cells, ACTH signalling of adrenocortical cells, IgE, FSH and hCG stimulation of granulosa cells, thyrotropin stimulation of thyroid cells, cell proliferation in the early developing ear and rat mammary gland. Consequently, IPGs or their antagonists can be used in the treatment or amelioration of disorders mediated by the growth factors or to mimic specific growth factor or hormone biological activities.

Examples of conditions which can be treated using IPGs or IPG antagonists include diabetes, obesity, dyslipidaemia, pre-eclampsia, neurotrophic disorders, hepatic damage and adrenal atrophy.

WO98/10791 discloses that pre-eclampsia is characterised by elevated levels of IPG-P and that it can be treated using an IPG-P antagonist. Compounds of the invention which are IPG-P antagonists, e.g. antagonists which compete with wild-type IPG-P but lack one or more of its activities, could be used in the treatment of pre-eclampsia.

The use of both IPG-P and IPG-A and IPG-A antagonists in the diagnosis and treatment of diabetes is disclosed in WO98/11435. This application discloses that in some forms of diabetes the ratio of P:A-type IPGs is imbalanced and can be corrected by administering a medicament containing an appropriate ratio of IPG-P, IPG-A or antagonist(s) thereof. In particular, it describes the treatment of obese type II diabetes (NIDDM) patients with a P-type IPG and/or an A-type IPG antagonist and the treatment of IDDM or lean type II diabetes (body mass index < 27) with a mixture of P- and A-type IPGs, typically in a P:A ratio of about 6:1 for males and 4:1 for females. The compounds and compositions of the present invention can be employed in such types of treatment. More particularly, the compounds are likely to be of use in the treatment of various form of diabetes and diabetic complications including diabetes due to insulin resistance, insulin resistance in type I diabetes and brittle diabetes, obese or lean type II diabetes, and of conditions associated with insulin resistance or insulin underproduction, such as neurotrophic disorders or polycystic ovary syndrome, lipodystrophy, age-related memory loss, and post-ischaemic damage secondary to stroke or post-transplant complications.

The compounds of this invention are also likely to be of use in controlling neuron proliferation or neurite outgrowth, either *in vitro* or *in vivo*, e.g. acting as a nerve or neurite growth factor mimetic second messenger. They may thus have applications in the treatment and/or diagnosis of any condition related to neuron proliferation or neurite differentiation. WO99/38516 discloses that IPG-A and synthetic mimetics thereof cause neuron proliferation, mimicking the action of the growth factor IGF-I. In contrast, IPG-P and synthetic mimetics thereof such as compound C4 cause neurite outgrowth. The neurons may be central (brain and spinal cord) neurons, peripheral (sympathetic, parasympathetic, sensory and enteric) neurons, e.g. the compounds used in the regeneration of peripheral nerves, or motor neurons. Treatments may involve the treatment of damage to nerve, spinal cord or central nervous system damage secondary to trauma, or autoimmune or metabolic damage, or post-ischaemic damage secondary to stroke or post-transplant complications, motor neuron disease, neurodegenerative disorders or neuropathy. Damage to the nervous system includes the results of trauma, stroke, surgery, infection (e.g. by viral agents), ischemia, metabolic disease, toxic agents, or a combination of these or similar causes. Motor neuron disease includes conditions involving spinal muscular atrophy, paralysis or amyotrophic lateral sclerosis. Neurodegenerative disorders include Parkinson's disease, Alzheimer's disease, epilepsy, multiple sclerosis, Huntingdon's chorea and Meniere's disease.

The compounds of the invention may also be useful as hepatocyte growth factor mimetic second messengers, e.g. in the preparation of medicaments for the treatment of hepatic damage caused by infection, alcohol abuse, drug sensitivity, or autoimmunity. The compounds may also be useful as fibroblast growth factor mimetic second messengers or epidermal growth factor mimetic second messengers, e.g. in the preparation of medicaments for the promotion of wound healing following surgery or trauma or tissue damage induced by ischaemia or autoimmunity.

In other embodiments, the compounds of the invention may be useful as adrenal cell growth factor mimetic second messengers or ACTH mimetic second messengers in the preparation of a medicament for the treatment of disease states involving adrenal atrophy.

The compounds of the invention can readily be tested using the assays identified herein to determine their suitability for some or all of the medical uses described above. Thus, even compounds with a relatively low activity in one of the enzymes assays disclosed herein, may be useful by virtue of possessing a different activity, and moreover the pattern of activities can be used to rapidly screen the compounds for suitability in the various medical applications disclosed herein.

| Activity | Diabetes I | Diabetes II | Obesity | Alzheimer's | Neurotrophics |
|---|---|---|---|---|---|
| PDH Kinase | Inhibit | Inhibit | No Effect | Inhibit | No effect |
| PDH Phosphatase | Activate | Activate | No effect | No effect | No effect |
| Acetyl CoA carboxylase I * | Activate | No effect | No effect | No effect | No effect |
| * found in liver and adipose cytosol. | | | | | |

Methods of Making the Compounds

Based on the disclosure herein, the knowledge in the art and in references[5-11], the skilled person could couple sugar residues and cyclitols together, optionally with one or more substituents. By way of example, compound RGL1119 was made in this way.

Useful guidance on the synthesis of the exemplified compounds and for introducing the substituents set out herein is provided by the papers by Gigg & Gigg, Khiar & Martin-Lomas[5] and Baeschlin et al[16] and the references cited therein.

Phosphoryl groups such as phosphate, cyclic phosphate or substituted phosphate or cyclic phosphate can be substituted into the compounds of the invention by the phosphate or phosphoramidite method, Bannwath et al, *Helvetica Chemica Acta,* 70:175-186, 1987 and Yu & Fraser-Reid, *Tetrahedron Lett.*, 29:979-982, 1988.

Phosphate protecting groups can also be synthesized according to the methods disclosed in Hoeben-Weyl, Methods of Organic Chemistry, volume 12/1 or 12/2, Teilheimer, Synthetic Methods of Organic Chemistry, Vol 45. Protecting groups for the OH of sugars include menthoxycarbonyl (MntCO), acetal (in particular, two R groups may together represent a bridging acetal such as *O*-cyclohexylidene, *O*-isopropylidene or *O*-benzylidene), *tert*-butyldimethylsilyl (TBDMS), benzyl (Bn), *tert*-butyldiphenylsilyl (TBDPS). Many protecting groups suitable for use in the syntheses and reactions of saccharides are known and are well documented in standard reference works. The choice depends in part on the route by which the compound is synthesised and/or on the uses to which it is to be put, including the reactions which it is subsequently intended to undergo.

## Bioactivity Assays

The compounds of the invention can be tested for one or more the characteristic IPG-P and/or IPG-A activities mentioned above to determine whether they will be suitable for use a IPG mimetics or antagonists. Preferred assays measure the effect of the compounds on PDH phosphatase, PKA or lipogenesis. Protocols for these assays are provided in Caro et al [14]. The compounds can also be tested to determine whether they activate or inhibit other enzymes involved in insulin signalling mechanism, such as glucose-6-phosphatase.

## Examples

### General Methods

All reactions were carried out under an atmosphere of dry argon using oven-dried glassware and freshly distilled and dried solvents. THF and diethyl ether were distilled from sodium benzophenone ketyl. Dichloromethane and acetonitrile were distilled from calcium hydride. TLC was performed on Silica gel $GF_{254}$ (Merck) with detection by charring with phosphomolibdic acid/EtOH. For flash chromatography, Silica Gel (Merck 230-400 mesh) was used. Columns were eluted with positive air pressure. Chromatographic eluents are given as volume to volume ratios (v/v). Routine NMR spectra were recorded with Bruker Avance DPX300 ($^1H$, 300 MHz), Bruker Avance DRX400 ($^1H$, 400 MHz), and Bruker Avance DRX500 ($^1H$, 500 MHz) spectrometers. Chemical shifts are reported in ppm, and coupling constants are reported in Hz. Spectra were referenced to the residual proton or carbon signals of the solvent. High-resolution mass spectra were recorded on a Kratos MS-80RFA 241-MC apparatus. Optical rotations were determined with a Perkin-Elmer 341 polarimeter. Elemental analyses were performed using a Leco CHNS-932 apparatus. The organic extracts were dried over anhydrous sodium sulfate and concentrated *in vacuo*.

The synthesis of compounds **1-4** involved the preparation of a glycosyl acceptor with position 6 differentiated to be reactive with the corresponding glycosyl donor. Protection of the pinitol unit as cyclohexane-1,2-diacetal as proposed by Ley [8] was attempted. The selectivity of the reaction of 5 with 1,1,2,2-tetramethoxycyclohexane arises from the stabilising influence of the four anomeric effects in the resulting acetal 6 and the equatorial arrangement of all four sterically demanding alkyl substituents of the central 1,4-dioxane unit (Scheme 1). Treatment of **6** with 2,2-dimethoxypropane in the presence of TsOH gave **7** in 80% yield.

Glycosylation of **7** with 2-azido-2-deoxy-3,4,6-tri-*O*-benzyl-D-glucopyransoltrichloroacetimidate **8** [9] prepared following a well established procedure[10,6b], in dichloromethane and using TMSOTf as promoter [11] gave a 2:1 mixture of the α **(9)** and β **(10)** linked pseudodisaccharides in 54% yield (Scheme 2). The acetal groups were removed [12] to give **11** and **12** respectively which were subjected to hydrogenolysis to afford finally **1** (RGL1024) and 2 (RGL1015) in quantitative yield.

Glycosylation of 7 with 2-azido-2-deoxy-3,4,6-tri-*O*-benzyl-D-galactopyranosc **(13)** under different conditions gave a mixture of α- and β-linked pseudodisaccharides **14** and 15 in moderate yield (Scheme 3). Removal of the acetal groups and subsequent hydrogenolysis afforded 3 (RGL1025) and 4 (RGL1119).

### 4,5-*O*-(1',2'-dimethoxycyclohexane-1',2'-diyl)-3-O-methyl-D-*chiro*-inositol (6)

3-*O*-methyl-D-*chiro*-inositol (D-pinitol) (283 mg, 1.457 mmol, 1 equiv) was dissolved in methanol (15 mL), then 1,2-cyclohexane diacetal (507 mg, 2.458 mmol, 1.7 equiv), trimethyl orthoformiate (200 mL, 1.752 mmol, 1.2 equiv) and 1-(*S*)-(+)-10-camphorsulfonic acid (24 mg, 0.102 mmol, 0.07 equiv) were added. The reaction mixture was heated at 70°C for 24 h whereupon it was diluted with MeOH and quenched with solid $NaHCO_3$. The residue was concentrated and purified by flash chromatography (Hex/EtOAc 1:15) to give 6 (222 mg, 0.664 mmol, 45%; 89% based on 134 mg of recovered D-pinitol). $R_f$ (Hex/EtOAc 1:20): 0.22; $[\alpha]^{20}_D$ -22.9 (c = 1.05, $CHCl_3$); $^1H$ NMR ($CDCl_3$, 300 MHz): d = 4.13 (Ys, 1H, $H_1$), 4.10 (s, 2H, $H_4$, $H_5$), 4.07 (Ys, 1H, $H_6$), 3.81 (dt, 1H, $J_{2-3}$ = 9.0 Hz, $J_{2-1}$ = 3.6 Hz, $J_{2-OH}$ = 1.8 Hz, $H_2$), 3.47 (t, 1H, $J$ = 9.0 Hz, $H_3$), 3.23 (s, 3H, $OCH_3$), 3.21 (s, 3H, $OCH_3$), 2.76 (d, 1H, $J_{OH-2}$ = 1.8 Hz, $OH_2$), 2.60 (s, 1H, $OH_1$), 2.56 (s, 1H, $OH_{-6}$), 1.86-1.78 (m, 1H, CDA), 1.77-1.66 (m, 3H, CDA), 1.57-1.50 (m, 2H, CDA), 1.42-1.34 (m, 2H, CDA); $^{13}C$-NMR ($CDCl_3$, 75 MHz); d = 98.9 (C), 98.0 (C), 80.2 ($C_3$), 71.5($C_2$), 70.8, 70.4, 70.3 ($C_1$, $C_4$, $C_6$), 68.7 ($C_5$), 61.1 ($OCH_3$), 47.0($OCH_3$, CDA), 46.8 ($OCH_3$, CDA), 27.1 ($CH_2$), 27.0 ($CH_2$), 21.5 ($CH_2$), 21.4 ($CH_2$); HRFABMS Calcd. for $C_{15}H_{26}O_8$: 357.3571 found: 357.1528; MALDI-TOF Calcd. for $C_{15}H_{26}O_8+Na^+$: 357.4, found: 357.5, calcd. for $C_{15}H_{26}O_8+K^+$: 373.5, found: 374.1

### 4,5-*O*-(1',2'-dimethoxycyclohexane-1',2'-diyl)-3-*O*-methyl-1,2-*O*-isopropyliden-D-*chiro*-inositol (7)

4,5-*O*-(1',2'-dimethoxycyclobexane-1',2'-diyl)-3-*O*-methyl-D-*chiro*-inositol (6) (326 mg, 0.975 mmol, 1 equiv) and 2,2-dimethoxypropane (1,321 mL, 1.072 mmol, 1.1 equiv) in acetone (4 mL) were treated with *p*-toluenesulfonic acid monohydrate (9.3 mg, 0.049 mmol, 0.05 equiv). The reaction was stirred for 1h, whereupon it was quenched with solid $NaHCO_3$, the solvent evaporated and the residue purified by flash chromatography (Hex/AcOEt 1:1) to give 7 (292 mg, 0.873 mmol, 81%) as a white solid. $R_f$(Hex/EtOAc 3:1): 0.71; $[\alpha]^{20}_D$ -18.0 (c = 0.30, $CHCl_3$); $^1H$ NMR ($CDCl_3$, 500 MHz): d = 4.27 (dd, 1H, $J_{1-2}$ = 7.3 Hz, $J_{1-6}$ = 3.2 Hz, $H_1$), 4.24 (t, 1H, $J$= 3.2 Hz, $H_6$), 4.16 (t, 1H, $J$ = 7.3 Hz, $H_2$), 4.06 (t, 1H, $J$ = 10.2 Hz, $H_4$), 3.94 (dd, 1H, $J_{5-4}$ = 10.2 Hz, $J_{5-6}$ = 3.2 Hz, $H_5$), 3.61 (s, 3H, $OCH_3$), 3.39 (dd, 1H, $J_{3-4}$ = 10.2 Hz, $J_{3-2}$ = 7.3 Hz, $H_3$), 3.23 (s, 3H, $OCH_3$, CDA), 3.22 (s, 3H, $OCH_3$, CDA), 1.86-1.78 (m, 1H, CDA), 1.77-1.66 (m, 3H, CDA),1.52 (s, 3H, $CH_3$), 1.57-1.51 (m, 2H, CDA), 1.36 (s, 3H, $CH_3$),1.41-1.33 (m, 2H, CDA); $^{13}C$-NMR ($CDCl_3$, 125 MHz): d =109.0 (C), 98.4 (C, CDA), 97.8 (C, CDA), 82.7 ($C_3$), 79.6 ($C_2$), 76.4 ($C_1$), 68.5 ($C_5$), 68.3 ($C_6$), 67,4($C_4$), 60.2 ($OCH_3$), 47.1 ($OCH_3$, CDA), 46.9 ($OCH_3$, CDA), 27.0 ($CH_3$), 27.1 ($CH_2$), 26.9 ($CH_2$), 26.0 ($CH_3$); 21.4 ($2CH_2$); Anal. Calcd. for $C_{18}H_{30}O_8$: C, 57.74%; H, 8.08%; found: 57.46%; H, 7.86%; HRFABMS Calcd. for $C_{18}H_{30}O_8+Na^+$: 397.1838, found: 397.1854; MALDI-TOF Calcd. for $C_{18}H_{30}O_8+Na^+$ : 397.4, found: 397.3 calcd. for $C_{18}H_{30}O_8+K^+$ : 413.5, found: 413.8

### Glycosylation reaction of 7 and 8

To a solution of **8** (809 mg, 1.305 mmol, 1.5 equiv) in $CH_2Cl_2$ (4 mL), compound 7 (295 mg, 0.788 mmol, 1 equiv) and freshly activated 4 Å molecular sieves were added and the mixture stirred for 1 h under Argon. Then, TMSOTf (12.6 mL, 0.104 mmol, 0.08 equiv) was added and the reaction mixture stirred for 24 h. The suspension was filtered through celite and the solvent evaporated under vacuum to provide a mixture of two disaccharides (α/β = 2:1) which can be separated by flash chromatography (Hex/EtOAc 4:1) to obtain **9** (235 mg, 0.282 mmol, 36%) and **10** (118 mg, 0.142 mmol, 18%).

### 2-Azido-2-deoxy-3,4,6-tri-*O*-benzyl-D-glucopyranosyl-α(1-6)-4,5-*O*-(1',2'-dimethoxycyclohexane-1',2'-diyl)-1,2-*O*-isopropyliden-3-*O*-methyl-D-*chiro*-inositol (9)

Rf (Hex/EtOAc 3:1): 0.17; $[\alpha]^{20}_D$ +48.8° (c = 0.25, $CHCl_3$); $^1H$ NMR ($CDCl_3$, 500 MHz): d = 7.43-7.22 (m, 15H, Ph), 4.94 (d, 1H, $J_{1'-2'}$ = 3.2 Hz, $H_{1'}$), 4.83 (d, 1H, $J$ = 13.0 Hz, CHPh), 4.82 (AB syst., 2H, $CH_2Ph$), 4.58 (d, 1H, $J$ = 12.0 Hz, CHPh), 4.58 (m, 1H, $H_{5'}$), 4.57 (d, 1H, $J$ = 13.0 Hz, CHPh), 4.42 (d, 1H, $J$= 12.0 Hz, CHPh), 4.25 (t, 1H, $J$ = 6.4 Hz,

$H_2$), 4.18 (t, 1H, *J* = 2.5 Hz, $H_1$), 4.16 (t, 1H, *J* = 2.5 Hz, $H_6$), 4.11 (t, 1H, *J* = 10.5 Hz, $H_4$), 3.96 (t, 1H, *J* = 10.1 Hz, $H_{3'}$), 3.90 (dd, 1H, $J_{5-4}$ = 10.5 Hz, $J_{5-6}$ = 2.5 Hz, $H_5$), 3.79 (t, 1H, *J* = 10.1 Hz, $H_{4'}$), 3.71 (dd, 1H, $J_{6a'-6b'}$ = 10.8 Hz, $J_{6a'-5'}$ = 2.4 Hz, $H_{6a'}$), 3.60 (s, 3H, $OCH_3$), 3.59 (dd, 1H, $J_{6a'-6b'}$ = 10.8 Hz, $J_{6b'-5'}$ = 1.6 Hz, $H_{6b'}$), 3.41 (dd, 1H, $J_{3-4}$ = 10.5 Hz, $J_{3-2}$ = 6.4 Hz, $H_3$), 3.36 (dd, 1H, $J_{2'-3'}$ = 10.2 Hz, $J_{2'-1'}$ = 3.2 Hz, $H_{2'}$), 3.19 (s, 6H, 2 $OCH_3$, CDA), 1.83-1.78 (m, 1H, CDA), 1.72-1.61 (m, 3H, CDA), 1.52 (s, 3H, $CH_3$), 1.55-1.43 (m, 2H, CDA), 1.40-1.30 (m, 2H, CDA), 1.36 (s, 3H, $CH_3$); MALDI-TOF Calcd. for $C_{45}H_{57}N_3O_{12}+Na^+$ : 855.0, found: 854.3, calcd. for $C_{45}H_{57}N_3O_{12}+K^+$ : 871.1, found: 870.5

**2-Azido-2-deoxy-3,4,6-tri-*O*-benzyl-D-glucopyranosyl-β(1-6)-4,5-*O*-(1',2'-dimethoxycyclohexane-1',2'-diyl)-1,2-*O*-isopropyliden-3-*O*-methyl-D-*chiro*-inositol (10)**

$R_f$(Hex/EtOAc 3:1): 0.20; $[\alpha]^{20}_D$ -8.0° (c = 1.18, $CHCl_3$); $^1H$ NMR ($CDCl_3$, 500 MHz): d 7.40-7.24 (m, 13H, Ph), 7.21-7.13 (m, 2H, Ph), 4.97 (d, 1H, $J_{1'-2'}$ = 8.1 Hz, $H_{1'}$), 4.92 (d, 1H, *J*= 10.8 Hz, CHPh), 4.82 ( d, 1H, *J*= 10.8 Hz, CHPh), 4.78 (d, 1H, *J* = 10.8 Hz, CHPh), 4.62 (d, 1H, *J* = 10.8 Hz, CHPh), 4.58 (AB syst., 2H, $CH_2Ph$), 4.49 (t, 1H, *J* = 2.5 Hz, $H_{o'}$), 4.30 ( dd, 1H, $J_{1-2}$ = 5.1 Hz, $J_{1-6}$ = 2.5 Hz, $H_1$), 4.20 (t, 1H, *J* = 10.8 Hz, $H_4$), 4.17 (t, 1H, *J* = 5.1 Hz, $H_2$), 3.98 (dd, 1H, $J_{5-4}$ = 10.8 Hz, $J_{5-6}$= 2.5 Hz, $H_5$), 3.71-3.57 (m, 3H, $H_{5'}$, $H_{6'}$, $H_{4'}$), 3.61 (s, 3H,$OCH_3$), 3.47-3.39 (m, 3H, $H_{3'}$, $H_3$, $H_{6'}$), 3.30 (dd, 1H, *J*= 8.1 Hz, $H_{2'}$), 3.23 (s, 3H, $OCH_3$, CDA), 3.18 (s, 3H, $OCH_3$, CDA), 1.83-1.77 (m, 1H, CDA), 1.80-1.43 (m, 5H, CDA), 1.52 (s, 3H, $CH_3$), 1.41-1.28 (m, 2H, CDA), 1.33 (s, 3H, $CH_3$); $^{13}C$-NMR ($CDCl_3$, 125 MHz): d = 138.5 (C), 138.4 (C), 138.3 (C), 128.9 (2CH), 128.8 (2CH), 128.8 (CH), 128.8 (2CH), 128.4 (2CH), 128.3 (2CH), 128.3 (CH), 128.2 (2CH), 128.1 (CH), 109.9 (C), 101.3 ($C_{1'}$), 98.4 (C, CDA), 98.0 (C, CDA), 83.9 ($C_{3'}$),83.6 ($C_3$),78.1 ($C_{4'}$),76.7 ($C_1$), 76.0 ($CH_2$),75.9 ($C_{6'}$),75.5 ($CH_2$),75.4 ($CH_2$),73.9 ($C_6$),72.6 ($C_{5'}$),68.8 ($C_5$),68.7 ($C_2$),68.1 ($C_4$), 67.5 ($C_{2'}$), 60.4 ($OCH_3$), 47.4 ($OCH_3$, CDA),47.3 ($OCH_3$, CDA),28.4 ($CH_3$),27.5 ($CH_2$),27.4 ($CH_2$),26.3 ($CH_3$),21.8 ($2CH_2$); HRFABMS Calcd. for $C_{45}H_{57}N_3O_{12}$ : 854.3840, found = 854.3872; MALDI-TOF Calcd. for $C_{45}H_{57}N_3O_{12}+Na^+$ : 855.0, found: 854.3, calcd. for $C_{45}H_{57}N_3O_{12}+K^+$ : 871.1, found: 870.4

**2-Azido-2-deoxy-3,4,6-tri-*O*-benzyl-D-galactopyranosyl-α(1-6)-4,5-*O*-(1',2'-dimethoxycyclohexane-1',2'-diyl)-1,2-*O*-isopropyliden-3-*O*-methyl-D-*chiro*-inositol (14)**

To a solution of 13 (84 mg, 0.14 mmol, 1.3 equiv), 7 (39 mg, 0.10 mmol, 1.0 equiv) in $CH_2Cl_2$:Hex, 1:3 (2 mL) and freshly activated 4 Å molecular sieves were added and the mixture stirred for 1 h under Argon. Then, TMSOTf (2.43 mL, 0.02 mmol, 0.15 equiv) was added at -40°C and the reaction mixture stirred for 24 h at 0°C. The suspension was filtered through celite and the solvent removed under vacuum to provide the crude material. Flash chromatography (Hex/EtOAc 4:1) afforded 14 (38 mg, 0.046 mmol, 46%). $R_f$(Hex/EtOAc 1:1): 0.67; $[\alpha]^{20}_D$ +59.7° (c = 1.44, $CHCl_3$); $^1H$ NMR ($CDCl_3$, 300 MHz) : d = 7.46-7.28, (m, 15H, Ph), 4.98 (d, 1H, *J*= 3.3 Hz, $H_{1'}$), 4.89 (d, 1H, *J*= 11.2 Hz, CHPh), 4.78 (d, 1H, *J*=11.1 Hz, CHPh), 4.69 (m, 1H, $H_{5'}$), 4.67 (d, 1H, *J*= 11.1 Hz, CHPh), 4.59 (d, 1H, *J*= 11.2 Hz, CHPh), 4.46, (AB syst., 2H, $CH_2Ph$),4.28 (t, 1H, *J*= 6.3 Hz, $H_2$), 4.20 (m, 2H, $H_1$, $H_6$), 4.15 (bs, 1H, $H_{4'}$), 4.11 (m, 1H, $H_4$), 4.04 (dd, 1H, $J_{3'-2'}$ = 10.6 Hz, $J_{3'-4'}$ = 2.4 Hz, $H_{3'}$), 3.92 (dd, 1H, $J_{4-5}$ = 10.8 Hz, $J_{5-6}$ = 2.1 Hz, $H_5$), 3.86 (dd, 1H, $J_{2'-3'}$ = 10.6 Hz, $J_{2'-1'}$ = 3.3 Hz, $H_{2'}$), 3.66-3.60 (m, 1H, $H_{6b'}$), 3.63 (s, 3H, $OCH_3$), 3.50 (dd, 1H, $J_{6a'-6b'}$ = 8.1 Hz, $J_{6a'-5'}$ = 5.4 Hz, $H_{6a'}$), 3.42 (dd, 1H, $J_{3-4}$ = 10.2 Hz, $J_{3-2}$ = 6.3 Hz, $H_3$), 3.20 (s, 3H, $OCH_3$, CDA), 3.18 (s, 3H, $OCH_3$, CDA), 1.90-1.63 (m, 4H, CDA), 1.54 (s, 3H, $CH_3$), 1.60-1.33 (m, 4H, CDA),1.40 (s, 3H, $CH_3$); $^{13}C$-NMR ($CDCl_3$, 75 MHz): δ 139.0 (C). 138.5 (C), 138.0 (C), 110.2 (C), 98.3 (C, CDA), 98.2 (C, CDA), 97.8 ($C_{1'}$), 83.6 ($C_3$), 80.6 ($C_2$), 77.2 ($C_{3'}$), 75.2 ($C_6$), 75.0 ($CH_2$), 73.9 ($C_{4'}$), 73.8 ($CH_2$), 72.8 ($C_1$), 72.7 ($CH_2$), 69.9 ($C_{5'}$), 68.4 ($C_{6'}$), 68.1 ($C_4$), 67.1 ($C_5$), 60.5 ($OCH_3$), 60.0 ($C_{2'}$), 47.2 ($2OCH_3$, CDA), 28.5 ($CH_3$), 27.4 ($CH_2$), 27.0 ($CH_2$), 26.7 ($CH_3$), 21.9 (2 $CH_2$); MALDI-TOF Calcd. for $C_{45}H_{57}N_3O_{12}+Na^+$ : 855.0 Found: 854.0 Calcd. for $C_{45}H_{57}N_3O_{12}+K^+$ : 871.1 Found: 870.1

**2-Azido-2-deoxy-3,4,6-tri-*O*-benzyl-D-glucopyranosyl-α(1-6)-3-*O*-methyl-D-*chiro*-inositol (11)**

Disaccharide (9) was dissolved in a mixture of trifluoroacetic acid/water (20:1, 3.7 mL) and stirred at room temperature for 40 min. Then, the reaction mixture was diluted with $CH_2Cl_2$ (10 mL) and immediately poured into an ice-cold, vigorously stirred solution of saturated aqueous sodium bicarbonate (90 mL). The layers were separated, and the aqueous phase extensively extrated ($CH_2Cl_2$, 4x 30 ml), dried over $Na_2SO_4$ and concentrated under vacuum. Purification by flash chromatography ($Cl_2CH_2$/MeOH, 11:1 and then $CH_2Cl_2$/MeOH, 15:1) afforded 11 (4 mg, 6.138 mmol, 56%). $R_f$ ($Cl_2CH_2$/MeOH, 9:1): 0.3; $[\alpha]^{20}_D$ +38.5° (c = 0.19, $CHCl_3$); $^1H$ NMR ($CDCl_3$, 500 MHz): d = 7.34-7.16 (m,15H, Ph), 4.94 (d, 1H, *J* = 3.5 Hz, $H_{1'}$), 4.84 (AB syst., 2H, $CH_2Ph$), 4.79 (d, 1H, *J*= 11.0 Hz, CHPh), 4.56 (d, 1H, *J*= 11.0 Hz, CHPh), 4.50 (d, 1H, *J* = 11.0 Hz, CHPh), 4.47 (d, 1H, *J* =11.0 Hz, CHPh), 4.16 (bs, 1H, $H_1$), 4.12 (m, 1H, $H_{5'}$), 4.04 (bs, 1H, $H_6$), 3.92 (m, 1H, $H_2$), 3.87-3.84 (m, 2H, $H_{3'}$, $H_5$), 3.71 (Yt, 1H, *J* = 8.0 Hz, $H_4$), 3.56 (m, 3H, $H_{4'}$, $2H_{6'}$), 3.63 (s, 3H, $OCH_3$), 3.44 (dd, 1H, $J_{2'-3'}$ = 10.0 Hz, $J_{1'-2'}$ = 3.5 Hz, $H_{2'}$), 3.37 (Yt, 1H, *J* = 8.0 Hz, $H_3$).

**2-Azido-2-deoxy-3,4,6-tri-*O*-benzyl-D-glucopyranosyl-β(1-6)-3-*O*-methyl-D-*chiro*-inositol (12)**

Compound **12** (28 mg, 43.0 mmol, 71%) was obtained via the general procedure described above for compound **11.** Purification by flash chromatography (Hex/EtOAc 1:20). $R_f$(EtOAc): 0.47; $^1$H NMR ($CDCl_3$, 500 MHz): d = 7.34-7.26 (m, 13H, Ph), 7.19-7.10 (m, 2H, Ph), 4.83 (AB syst., 2H, $CH_2$Ph), 4.78 (d, 1H, *J* = 11.0 Hz, CHPh), 4.54 (AB syst., 2H, $CH_2$Ph), 4.52 (d, 1H, *J* = 11.0 Hz, CHPh), 4.46 (d, 1H, *J* = 8.0 Hz, $H_{1'}$), 4.21 (bs, 1H, $H_1$), 4.10 (bs, 1H, $H_6$), 3.94-3.90 (m, 2H, $H_2$, $H_5$), 3.80 (t, 1H, *J* = 8.5 Hz, $H_4$), 3.69-3.59 (m, 2H, $2H_{6'}$), 3.62 (s, 3H, $OCH_3$), 3.58 (t, 1H, *J*= 9.5 Hz, $H_{4'}$), 3.47 (t, 1H, *J* = 9.5 Hz, $H_{3'}$), 3.46-3.36 (m, 3H, $H_{2'}$, $H_{5'}$, $H_3$), 3.35-3.27 (bs, 1H, OH), 3.27-3.15 (bs, 1H, OH), 3.15-2.94 (2bs, 2H, 2OH).

**2-Azido-2-deoxy-3,4,6-tri-*O*-benzyl-D-galactopyranosyl-α(1-6)-3-*O*-methyl-D-*chiro*-inositol (16)**

Compound **16** (7 mg, 10.7 mmol, 41 %) was obtained *via* the general procedure described above for compound 11, using purification by flash chromatography ($CH_2Cl_2$/MeOH, 10:1). $R_f$ ($CH_2Cl_2$/MeOH 9:1): 0.48; $^1$H NMR ($CDCl_3$, 500 MHz): d = 7.36-7.21 (m, 15H, Ph), 4.96 (d, 1H, $J_{1\text{-}2}$ = 3.5 Hz, $H_{1'}$), 4.85 (d, 1H, *J* = 11.5 Hz, CHPh), 4.71 (d, 1H, *J*= 11.0 Hz, CHPh), 4.67 (d, 1H, *J* = 11.0 Hz, CHPh), 4.49 (d, 1H, *J* = 11.0 Hz), 4.49 (d, 1H, *J* = 11.5 Hz, CHPh), 4.40 (d, 1H, *J* = 11.0 Hz, CHPh), 4.20 (dd, 1H, $J_{4'\text{-}5'}$ = 7.5 Hz, $J_{5'\text{-}6'}$ = 4.5 Hz, $H_{5'}$), 4.15 (t, 1H, *J*= 4.0 Hz, $H_1$), 4.01 (t, 1H, *J* = 4.0 Hz, $H_6$), 3.95-3.90 (m, 2H, $H_{2'}$, $H_{4'}$), 3.87 (m, 1H, $H_2$), 3.84 (dd, $J_{2'\text{-}3}$'= 10.5 Hz, $J_{3'\text{-}4'}$ = 2.5 Hz, $H_{3'}$), 3.82-3.77 (m, 1H, $H_5$), 3.65 (t, 1H, *J* = 8.2 Hz, $H_4$), 3.62 (s, 3H, $OCH_3$), 3.58 (dd, 1H, $J_{6a'\text{-}6b'}$ = 9.5 Hz, $J_{5'\text{-}6a'}$ = 7.5 Hz, $H_{6a'}$), 3,38 (dd, 1H, $J_{6a'\text{-}6b'}$ = 9.5 Hz, $J_{5'\text{-}6b'}$ = 4.5 Hz, $H_{6b'}$), 3.35 (t, 1H, J= 8.2 Hz, $H_3$).

**2-Amino-2-deoxy-D-glucopyranosyl-α(1-6)-3-*O*-methyl-D-*chiro*-inositol (1)**

Compound 11 (3.8 mg, 5.831 mmol, 1.0 equiv) and 10% Pd/C (10 mg, 9.398 mmol) were stirred in methanol under a hydrogen atmosphere for 24 h. The slurry was filtered, washed with water and the filtrate was concentrated and lyophilized to give the fully deprotected disaccharide 1 (2.6 mg, 7.3 mmol, quantitative). $R_f$ (EtOAc/MeOH/$H_2O$/AcOH 2:2:1:1): 0.44;$[\alpha]^{20}_D$ +55.2° (c = 0.125, $H_2O$). $^1$H NMR ($D_2O$, 500 MHz): δ = 5.22 (d, 1H, $H_{1'}$), 4.16 (t, 1 H, $H_1$), 4.19 (t, 1H, $H_6$), 4.07 (dt, 1H, $H_{5'}$), 3.90 (dd, 1H, $H_5$), 3.85-3.75 (m, 3H, $H_{4'}$, $H_{6a'}$, $H_{6b'}$), 3.80 (dd, 1H, $H_2$), 3.70 (t, 1H, $H_{3'}$), 3.64 (s, 3H, $OCH_3$), 3.52 (t, 1H, $H_4$), 3.36 (t, 1H, $H_3$), 3.24 (dd, 1H, $H_{2'}$).

**2-Amino-2-deoxy-D-glucopyranosyl-β(1-6)-3-*O*-methyl-D-*chiro*-inositol (2)**

Compound 12 (24 mg, 67.54 mmol, 98%) was treated as described above to afford compound 2. $R_f$(EtOAc/MeOH/$H_2O$/AcOH 2:2:1:1): 0.42, $^1$H NMR ($D_2O$, 500 MHz): d = 4.70 (d, 1H, *J* = 8.5 Hz, $H_{1'}$), 4.32 (Yt, 1H, *J* = 3.5 Hz, $H_1$), 4.12 (Yt, 1H, *J* = 3.5 Hz, $H_6$), 3.96 (dd, 1H, $J_{6a'\text{-}6b'}$ =9.5 Hz, $J_{6a'\text{-}5'}$ = 1.7 Hz, $H_{6a'}$), 3.92 (dd, 1H, $J_{5\text{-}4}$ = 8.5 Hz, $J_{5\text{-}6}$ = 3.5 Hz, $H_5$), 3.88 (dd, 1H, $J_{2\text{-}3}$ = 9.8 Hz, $J_{1\text{-}2}$= 6.6 Hz, $H_2$), 3.82 (dd, 1H, $J_{6a'\text{-}6b'}$ = 9.5 Hz, $J_{5'\text{-}6b'}$ = 4.0 Hz, $H_{6b'}$), 3.78 (t, 1H, *J* = 9.5 Hz, $H_{4'}$), 3.66 (s, 3H, $OCH_3$), 3.48 (t, 1H, *J* = 9.8 Hz, $H_{4'}$), 3.57-3.51 (m, 2H, $H_{3'}$, $H_{5'}$), 3.40 (t, 1H, *J* = 9.8 Hz, $H_3$), 2.86 (t, 1 H, *J* = 8.5 Hz, $H_{2'}$).

**2-Amino-2-deosy-D-glucopyranosyl-α(1-6)-3-*O*-methyl-D-*chiro*-inositol (14)**

Compound 14 (5.3 mg, 14.9 mmol, quantitative) was obtained *via* the general procedure described above for compound 12. $R_f$ ($Cl_2CH_2$/MeOH 9/1): 0.071; $[\alpha]^{20}_D$ (c = 0.305, $H_2O$): +70.1; $^1$H NMR ($D_2O$, 500 MHz): δ = 5.31 (d, 1H, *J*= 3.5 Hz, $H_{1'}$), 4.29 (t, 1H), 4.19 (Ψt, 1H, $H_1$), 4.14 (Ψt, 1H, $H_6$), 4.05 (Ψs, 1H, $H_{4'}$), 3.92 (dd, 1H), 3.77 (d, 3H), 3.72 (t, 1H), 3.65 (s, 3H, $OCH_3$), 3.62 (m, 1H), 3.52 (dd, 1H, $J_{2'\text{-}3'}$ = 11.0 Hz, $J_{1'\text{-}2'}$= 3.5 Hz, $H_{2'}$), 3.37 (t, 1H, *J* = 10.0 Hz, $H_3$).

**2-Deoxy-3,4,6-tri-*O*-acetyl-2-trichloroacetamide-D-galactopyranosyl-β-(1-6)-1,2-diisopropylidene-3,4-(1', 2'-dimethoxycyclohexane-1',2'-diyl)-3-*O*-methyl-D-*chiro*-inositol (3) (Synthesis of RGL1119, Scheme 4)**

A 10 mL round-bottomed flash was charged with donor 1 (81 mg, 0.14 mmol, 1.6 eq.), acceptor **2** (32 mg, 0.08 mmol, 1.0 eq), freshly 4 Å molecular sieves and $CH_2Cl_2$ (2 mL) and the mixture was stirred for 1 h under Argon. Then TfOTMS was added (3.1 mL, 0.02 mmol, 0.2 eq) at 0°C and the reaction mixture was stirred for 5 h. The suspension was filtered through a short pad of celite and the solvent removed to provide the crude material. Flash chromatography (Hex/EtOAc 2:1) afforded 9b (42 mg, 0.054 mmol, 63%). Rf(Hex/EtOAc 1:3): 0.52; $[\alpha]_D^{20}$-27.5 (c = 1.19, $CHCl_3$); $^1$H NMR ($CDCl_3$, 500 MHz) : δ = 6.59 (d, 1H, $J_{NH\text{-}H2'}$- 9.0 Hz, NH), 5.35 (d, 1H, J = 2.8 Hz, $H_{4'}$), 5.28 (d, 1H, J = 8.0 Hz, $H_{1'}$), 5.16 (dd, 1H, $J_{H3'\text{-}H2'}$= 11.0 Hz, $J_{H3'\text{-}H4'}$=2.8 Hz, $H_{3'}$), 4.46 (s, 1H, $H_6$), 4.26 (m, 1H, $H_1$),4.20-4.10 (m, 3H, $H_{2'}$, $H_{6a'}$, $H_{6b'}$), 4.07 (t, 1H, J = 6.0 Hz, $H_2$), 4.02 (t, 1H, J =10.0 Hz, $H_4$), 3.95 (m, 1H, $H_5$), 3.85 (m, 1H, $H_{5'}$), 3.53 (s, 3H, $OCH_3$), 3.33 (t, 1H, J = 10.0 Hz, $H_3$), 3.22 (s, 3H, $OCH_3$, CDA), 3.20 (s, 3H, $OCH_3$, CDA), 2.16 (s, 3H, $CH_3CO$), 2.02 (s, 3H,

$CH_3CO$), 1.98 (s, 3H, $CH_3CO$), 1.72-1.60 (m, 4H, $2CH_2$), 1.52-1.48 (m, 2H, $CH_2$), 1.51 (s, 1H, $CH_3$), 1.36-1.31 (m, 2H, $CH_2$), 1.34 (s, 3H, $CH_3$); $^{13}C$ NMR ($CDCl_3$, 125 MHz): $\delta$ = 170.7 ($CH_3\underline{C}O$), 170.3 ($CH_3\underline{C}O$), 170.2 ($CH_3\underline{C}O$), 162.2 ($CCl_3\underline{C}O$), 109.6 ($\underline{C}Cl_3CO$), 99.4 ($C_{1'}$), 97.9 (C, CDA), 97.8 (C, CDA), 92.2 (C), 83.5 ($C_3$), 79.6 ($C_2$), 76.0 ($C_1$), 72.2 ($C_6$), 71.2 ($C_{5'}$), 70.1 ($C_{3'}$), 68.2 ($C_5$), 67.7($C_4$), 66.8 ($C_{4'}$), 61.4 ($C_{6'}$), 59.8 ($OCH_3$), 53.1 ($C_{2'}$), 47.3 ($OCH_3$, CDA), 46.9 ($OCH_3$, CDA), 28.5 ($2CH_2$), 27.2 ($CH_3$), 27.0 ($CH_3$), 26.2 ($2CH_2$), 21.4 ($\underline{C}H_3CO$), 20.7 ( $\underline{C}H_3CO$), 20.6 ($\underline{C}H_3CO$).

**2-Deoxy-2-trichloroacetamido-3,4,6-tri-*O*-acetyl-D-galactopyranosyl-β-(1-6)-3-*O*-methyl-D-*chiro*-inositol(4)**

Pseudodisaccharide 3 (36 mg, 0.05 mmol, 1.0 eq) was dissolved in a mixture of trifluoroacetic acid/water (15: 1, 3.2 mL) and stirred for 3 h at room temperature. The solvent was removed under vacuum and the crude was purified by flash chromatography to provide 4 (22 mg, 0.03 mmol, 75%) as a white solid. Rf ($Cl_2CH_2$/MeOH 7:1): 0.15; $[\alpha]_D^{20}$-1.7 (c = 0.72, MeOH); $^1H$ NMR (MeOD, 500 MHz) : $\delta$ = 5.36 (d, 1H, $J_{H4'-H5'}$= 3.5 Hz, $H_{4'}$), 5.24 (dd, 1H, $J_{H3'-H2'}$= 11.1 Hz, $J_{H3'-H4'}$= 3.5 Hz $H_{4'}$), 5.01 (d, 1H, J = 8.5 Hz, $H_{1'}$), 4.16 (m, 2H, $H_{6a'}$, $H_{6b'}$), 4.08 (dd, 1H, $J_{H2'-H3'}$= 11.2 Hz, $J_{H2'-H1'}$= 8.5 Hz, $H_{2'}$), 4.05-4.02 (m, 2H, $H_{5'}$, $H_6$), 3.98 (t, 1H, J= 3.0 Hz, $H_1$), 3.79 (dd, 1H, $J_{H5-H4}$= 9.5 Hz, $J_{H5-H6}$= 3.0 Hz, $H_5$), 3.70 (dd, 1H, $J_{H2-H3}$= 9.7 Hz, $J_{H2-H1}$= 3.0 Hz, $H_2$), 3.58 (m, 1H, $H_4$), 3.56 (s, 3H. $OCH_3$), 3.22 (t, 1H, J = 9.7 Hz, $H_3$), 2.16 (s, 3H, $\underline{C}H_3CO$), 2.05 (s, 3H, $CH_3CO$), 1.93 (s, 3H, $CH_3CO$); $^{13}C$ NMR (MeOD, 125 MHz) : $\delta$ = 170.8 ($CH_3\underline{C}O$), 170.6 ($CH_3\underline{C}O$), 170.1 ($CH_3\underline{C}O$), 163.2 ($Cl_3C\underline{C}O$), 101.3 ($C_{1'}$), 92.5 ($Cl_3\underline{C}CO$), 83.3 ($C_3$), 79.1 ($C_6$), 72.4 ($C_4$), 71.4 ($C_1$), 71.0 ($C_5$), 70.6 ($C_{5'}$), 70.4 ($C_2$), 70.3 ($C_{3'}$), 66.9 ($C_{4'}$), 61.3 ($C_{6'}$), 58.9 ($OCH_3$), 52.5 ($C_{2'}$), 19.2, 19.1, 19.0 ($3\underline{C}H_3CO$); anal. calcd. for $C_{21}H_{30}NO_{14}Cl_3$: C, 40.24%; H, 4.82%; N, 2.23%; found: 38.89%; H, 5.17%; N, 1.94%.

**2-amino-2-deoxy-D-galactopyranosyl-β-(1-6)-3-*O*-methyl-D-*chiro*-inositol (5, RGL 1119)**

Compound **4** (6 mg, 10 mmol) and $Ba(OH)_2$ (8 mg, 0.094 mmol, 4.8 eq.) was stirred in water/EtOH 1:1 (4 mL) for 1 h at 90°C, whereupon the solvent was removed under vacuum. Cold water was added to the residue and filtered through a paper filter to eliminate $Ba(OH)_2$.. The filtrate was removed under vacuum and the residue was redissolved in the minimum amount of MeOH and loaded onto a Varian CBA (carboxylic acid) cartridge (3 $cm^3$/500 mg) preequilibrated with MeOH. After elution with some lengths of MeOH to remove barium salts, pseudodisaccharide was neutralized loading it directly onto a Varian PSA (ethylenediamine-N-propyl) cartridge preequilibrated with MeOH to give RGL 1119 (3 mg, 8 mmol, 79%). $R_f$ (EtOAc/MeOH/$H_2O$/AcOH 2:2:1:1): 0.29; $[\alpha]_D^{20}$ +8.1 (c = 0.28, $H_2O$); $^1H$ NMR ($D_2O$, 500 MHz): $\delta$ = 4.32 (d, 1H, J = 9.0 Hz, $H_{1'}$), 4.15 (bt, 1H, J = 3.5 Hz, $H_6$), 3.90 (bt, 1H, J = 3.5 Hz, $H_1$), 3.75-3.71 (m, 3H, $H_5$, $H_2$, $H_{4'}$), 3.68-3.53 (m, 4H, $H_{6a'}$, $H_{6b'}$, $H_{5'}$, $H_4$), 3.47 (s,3H, $OCH_3$), 3.42 (dd, 1H, $J_{H3'-H2'}$ = 10.0 Hz, $J_{H3'-H4'}$ = 3.5 Hz, $H_{3'}$), 3.21 (t, 1H, J = 9.5 Hz, $H_3$), 2.74 (t, 1H, J = 9.0 Hz $H_{2'}$); $^{13}C$ NMR ($CDCl_3$, 125 MHz) : $\delta$ = 100.8 ($C_{1'}$), 83.5 ($C_3$), 82.2 ($C_1$), 75.9 ($C_4$), 73.9 ($C_{3'}$), 73.1 ($C_{5'}$), 71.3 ($C_6$), 70.8 ($C_2$), 70.0 ($C_5$), 68.2 ($C_{4'}$), 62.4 ($C_{6'}$), 53.7 ($C_{2'}$). CI HRMS calcd. for $C_{13}H_{25}NO_{10}$($M^+$+H): 356.1556, found: 356.1548.

**Assay Data**

**PDH % activation:**

| | **100μM** |
|---|---|
| RGL1015 | 38% |
| RGL1024 | 65% |
| RGL1025 | 17% |

**PKA % inhibition:**

| | **0.1μM** | **1μM** | **10μM** |
|---|---|---|---|
| RGL1015 | 14% | 15% | - |
| RGL1024 | 17% | 34% | 2% |
| RGL1025 | 48% | -13% | - |

Figure 1 shows a graphical comparison of PDH phosphatase activation at 100μM of compounds of the invention as compared to D-pinitol. In particular, preferred compounds having 1,6 linkages are good activators of PDH phosphatase activity.

**References:**

[1] (a) Varela-Nieto et al, *Comp. Biochem. Physiol.*,. 115B:223-241, 1996; (b) Strälfors, *Bioassays,* 19:327-335, 1997; (c) Field, *Glycobiology,* 7:161-169, 1997; (d) Jones & Varela-Nieto, *Int. J. Biochem. Cell Biol.,* 30:313-326, 1998.

[2] Mato et al, *Biochem. Biophys. Res. Commun.,* 146:746-770, 1987.

[3] Larner et al, *Biochem. Biophys. Res. Commun.*, 151:1416-1426, 1998.

[4] Caro et al, *Biochem. Mol. Med,* 61:214-228, 1997.

[5] For reviews on the synthesis of these structures see: (a) Gigg & Gigg in *Glycopeptides and Related Compounds,* Large & Warren, Eds., Marcel Dekker, New York, 1997, pp 327-392; (b) Khiar & Martin-Lomas in *Carbohydrate Mimics.* Concepts and Methods, Chapleur Ed Wiley VCH, 1998, pp 443-462; (c) Dietrich et al, *Chem. Eur. J.*, 5: 320-336, 1999.

[6] Jaramillo et al, *J. Org. Chem.*, 59, 3135-3141, 1994.

[7] Corey & Venkateswarlu, *J. Am. Chem. Soc.*, 94:6190, 1974.

[8] Ley et al, Angew. *Chem. Int. Ed. Engl.*, 33:2290-2292, 1994.

[9] Kinzi & Schmidt, *Liebigs Ann. Chem.*, 1537-1545, 1985,

[10] Vasella et al, *Helv. Chim. Acta.*, 74:2073-2077, 1991.

[11] Schmidt & Kinzi, *Adv. Carbohydy Chem. Biochem.*, 50:21-123, 1994.

[12] Once et al, *Chem. Eur. J.,* 3:431-440, 1997.

[13] Rademacher et al, *Brazilian J. Med Biol. Res.,* 27:327-341, 1994.

[14] Caro et al, *Biochem. Molec. Med.*, 61:214-228, 1997.

[15] Kunjara et al, In: Biopolymers and Bioproducts: Structure, Function and Applications, Ed Svati et al, 301-305, 1995.

[16] Baeschlin et al, *Chem. Eur. J.,* 6(1):172-186,2000.

WO98/11116 and WO98/11117 (Rademacher Group Limited).

WO98/11435 and WO98/10791 (Rademacher Group Limited).

WO99/38516 (Rademacher Group Limited).

## Claims

**1.** A compound represented by the general formula:

**X- 1,6-pinitol**

wherein:

X represents a sugar residue;

the sugar residue is substituted with between one, two or three groups, and the pinitol is unsubstituted or is further substituted with between one and four groups, the group or groups being independently selected from:

(a) a phosphoryl group which is phosphate $-O-P(O)(OH)_2$; thiophosphate $-OP(S)(OH)_2$; a phosphate ester $-O-P(O)(OR)_2$; a thiophosphate ester $-OP(S)(OR)_2$; phosphonate -O-P(O)OHR; thiophosphonate -O-P(S)OHR; substituted phosphonate $-O-P(O)OR_1R_2$; substituted thiophosphonate $-OP(S)OR_1R_2$; -O-P(S)(OH)(SH); or cyclic phosphate $-O-P(O)(OR)_2$;

(b) a phosphorus containing group which is phosphoramidite $-O-P(OR)-NR_1R_2$ or phosphoramidate $-O-P(O)(O^-)-NR_1R_2$;

(c) a sulphur group which is $-O-S(O)(OH)_2$, -SH, -SR, -S(→O)-R, $-S(O)_2R$, $RO-S(O)_2$, $-O-SO_2NH_2$, $-O-SO_2R_1R_2$ or sulphamide $-NHSO_2NH_2$;

(d) an amino group which is -NHR, $-NR_1R_2$, -NHAc, -NHCOR, -NH-O-COR, - $NHSO_3^-$, or $-NHSO_2R$, $-N(SO_2R)$, or an amidino group which is $-NH-C(=NH)NH_2$ or an ureido group which is $-NH-CO-NR_1R_2$ or a thiouriedo group which is $-NH-C(S)-NH_2$;

(e) a hydroxy group or a substituted hydroxy groups represented by $-OR_3$, where $R_3$ is $C_{1-10}$ unsubstituted

or substituted alkyl, $CHF_2$, $CF_3$, alkoxyalkyl, aryloxyalkyl, cycloalkyl, alkenyl (unsubstituted alkyl), alkylene ($C_{3-7}$ cycloalkyl), -OCOR, aryl, heteroaryl or acetal, or where two hydroxyl groups are joined as a ketal;
(f) a halogen substituent such as fluorine or chlorine;
(g) hydrogen to provide a deoxy sugar.

wherein R, $R_1$ and $R_2$ are independently hydrogen or $C_{1-10}$ unsubstituted or substituted alkyl or aryl; or a derivative thereof.

**2.** The compound of claim 1, wherein the sugar residue is a hexose or a pentose, or substituted forms thereof.

**3.** The compound of claim 2, wherein the sugar residue is a hexose selected from the group consisting of glucose, galactose or mannose.

**4.** The compound of claim 2, wherein the sugar residue is a hexosamine.

**5.** The compound of claim 4, wherein the hexosamine is galactosamine or glucosamine.

**6.** The compound of any one of the preceding claims, wherein the sugar residue and the cyclitol are α-linked.

**7.** The compound of any one of claims 1 to 5, wherein the sugar residue and the cyclitol are β-linked.

**8.** The compound of any one of the preceding claims which is selected from the group consisting of:

*O*-(2-amino-2-deoxy-D-glucopyranosyl)-β(1,6)-D-3-*O*-methyl-*chiro*-inositol;
*O*-(2-amino-2-deoxy-D-glucopyranosyl)-α(1,6)-D-3-*O*-methyl-*chiro*-inositol;
*O*-(2-amino-2-deoxy-D-galactopyranosyl)-α(1,6)-D-3-*O*-methyl-*chiro*-inositol; and
1'-D-6-*O*-(2'-amino-2'-deoxy-β-D-galactopyranosyl)-3-*O*-methyl-D-*chiro*-inositol; or a derivative thereof.

**9.** The compound of any one of the preceding claims, wherein the derivative is a salt, a coordination complex with a metal ion, an ester, a free acid or base, a hydrate or a protecting group.

**10.** A composition comprising a compound of any one of the preceding claims, in combination with a pharmaceutically acceptable carrier.

**11.** A compound of any one of claims 1 to 9 for use in therapy.

**12.** Use of a compound of any one of claims 1 to 9 for the preparation of a medicament for the treatment of a condition ameliorated by the administration of an inositol phosphoglycan (IPG) second messenger or an IPG antagonist.

**Patentansprüche**

**1.** Verbindung der allgemeinen Formel:

X-1,6-Pinitol

worin:

X für einen Zuckerrest steht;
der Zuckerrest mit einer, zwei oder drei Gruppen substituiert ist und das Pinitol unsubstituiert ist oder weiter mit einer bis vier Gruppen substituiert ist, wobei die Gruppe oder Gruppen unabhängig voneinander ausgewählt ist/sind aus:

(a) einer Phosphorylgruppe, die Phosphat $-O-P(O)(OH)_2$; Thiophosphat $-O-P(S)(OH)_2$; ein Phosphatester $-O-P(O)(4R)_2$; ein Thiophosphatester $-O-P(S)(OR)_2$; Phosphonat -O-P(O)OHR; Thiophosphonat -O-P(S)OHR; substituiertes Phosphonat $-O-P(O)OR_1R_2$; substituiertes Thiophosphonat $-O-P(S)OR_1R_2$; -O-P(S)

(OH)(SH); oder zyklisches Phosphat -O-P(O)(OR)$_2$ ist;

(b) einer phosphorhältigen Gruppe, die Phosphoramidit -O-P(OR)NR$_1$R$_2$ oder Phosphoramidat -O-P(O)(O$^-$)-NR$_1$R$_2$ ist;

(c) einer Schwefelgruppe, die -O-S(O)(OH)$_2$, -SH, -SR, -S(→O)-R, -S(O)$_2$R, RO-S(O)$_2^-$, -O-SO$_2$NH$_2$, -O-SO$_2$R$_1$R$_2$ oder Sulfamid -NHSO$_2$NH$_2$ ist;

(d) einer Aminogruppe, die -NHR, -NR$_1$R$_2$, -NHAc, -NHCOR, -NH-O-COR, -NHSO$_3^-$ oder -NHSO$_2$R ist, oder einer Amidinogruppe, die -NH-C(=NH)NH$_2$ ist, oder einer Ureidogruppe, die -NH-CO-NR$_1$R$_2$ ist, oder einer Thioureidogruppe, die -NH-C(S)-NH$_2$ ist;

(e) einer Hydroxygruppe oder substituierten Hydroxygruppen, die durch -OR$_3$ dargestellt sind, worin R$_3$ ein unsubstituiertes oder substituiertes C$_{1-10}$-Alkyl, CHF$_2$, CF$_3$, Alkoxyalkyl, Aryloxyalkyl, Cycloalkyl, Alkenyl (unsubstiutiertes Alkyl), Alkylen (C$_{3-7}$-Cycloalkyl), -OCOR, Aryl, Heteroaryl oder Acetal ist oder worin zwei Hydroxygruppen als Ketal verbunden sind;

(f) einem Halogensubstituenten, wie z.B. Fluor oder Chlor;

(g) Wasserstoff, um einen Desoxyzucker bereitzustellen;

worin R, R$_1$ und R$_2$ unabhängig voneinander Wasserstoff oder ein unsubstituiertes oder substituiertes C$_{1-10}$-Alkyl oder Aryl sind;
oder ein Derivat davon.

2. Verbindung nach Anspruch 1, worin der Zuckerrest eine Hexose oder eine Pentose ist, oder substituierte Formen davon.

3. Verbindung nach Anspruch 2, worin der Zuckerrest eine Hexose ist, die aus der aus Glucose, Galactose und Mannose bestehenden Gruppe ausgewählt ist.

4. Verbindung nach Anspruch 2, worin der Zuckerrest Hexosamin ist.

5. Verbindung nach Anspruch 4, worin das Hexosamin Galactosamin oder Glucosamin ist.

6. Verbindung nach einem der vorangegangenen Ansprüche, worin der Zuckerrest und der Cyclit durch eine α-Bindung verbunden sind.

7. Verbindung nach einem der Ansprüche 1 bis 5, worin der Zuckerrest und der Cyclit durch eine β-Bindung verbunden sind.

8. Verbindung nach einem der vorangegangenen Ansprüche, ausgewählt aus der aus
O-(2-Amino-2-desoxy-D-glucopyranosyl)-β(1,6)-D-3-O-methyl-chiro-inosit;
O-(2-Amino-2-desoxy-D-glucopyranosyl)-α(1,6)-D-3-O-methyl-chiro-inosit;
O-(2-Amino-2-desoxy-D-galactopyranosyl)-α(1,6)-D-3-O-methyl-chiro-inosit; und
1'-D-6-O-(2'-Amino-2'-desoxy-β-D-galactopyranosyl)-3-O-methyl-D-chiro-inosit; bestehenden Gruppe, oder ein Derivat davon.

9. Verbindung nach einem der vorangegangenen Ansprüche, worin das Derivat ein Salz, ein Koordinationskomplex mit einem Metallion, ein Ester, eine freie Säure oder Base, ein Hydrat oder eine Schutzgruppe ist.

10. Zusammensetzung, umfassend eine Verbindung nach einem der vorangegangenen Ansprüche in Kombination mit einem pharmazeutisch annehmbaren Träger.

11. Verbindung nach einem der Ansprüche 1 bis 9 zur Verwendung in einer Therapie.

12. Verwendung einer Verbindung nach einem der Ansprüche 1 bis 9 zur Herstellung eines Medikaments zur Behandlung eines Leidens, das durch die Verabreichung eines zweiten Inositphosphoglycan- (IPG-) Messengers oder eines IPG-Antagonisten gelindert wird.

## Revendications

1. Un composé représenté par la formule générale:

X-1,6-pinitol

où:

X représente un résidu de sucre;
le résidu de sucre est substitué par entre un, deux ou trois groupes, et le pinitol n'est pas substitué ou est encore substitué par entre un et quatre groupes, le ou les groupes étant indépendamment sélectionnés parmi:

(a) un groupe phosphoryle qui est phosphate -O-P(O) $(OH)_2$; thiophosphate -O-P(S) $(OH)_2$; un phosphate ester -O-P(O)$(OR)_2$; un thiophosphate ester -O-P(S)$OR_2$; phosphonate -O-P(O)OHR; thiophosphonate -O-P(S)OHR; phosphonate substitué -O-P(O)$OR_1R_2$; thiophosphonate substitué -O-P(O)$OR_1R_2$; -O-P(S) (OH) (SH) ; ou phosphate cyclique -O-P(O)$(OR)_2$;
(b) un groupe contenant du phosphore qui est phosphoramidite -O-P(OR)-$NR_1R_2$ ou phosphoramidate -O-P(O) (O) -$NR_1R_2$;
(c) un groupe contenant du soufre qui est -O-S(O) $(OH)_2$, -SH, -SR, -S(→O)-R, -S$(O)_2$R, RO-S$(O)_2^-$, -O-$SO_2NH_2$, -O-$SO_2R_1R_2$ ou sulphamide -$NHSO_2NH_2$;
(d) un groupe amino qui est -NHR, -$NR_1R_2$, -NHAc, -NHCOR, -NH-O-COR, -$NHSO_3^-$, ou -$NHSO_2R$, -N ($SO_2R$), ou un groupe amidino qui est -NH-C(=NH)$NH_2$ ou un groupe ureido qui est -NH-CO-$NR_1R_2$ ou un groupe thioureido qui est -NH-C(S)-$NH_2$;
(e) un groupe hydroxy ou un groupe hydroxy substitué représenté par -$OR_3$, où $R_3$ est un alkyle nonsubstitué ou substitué en $C_{1-10}$, $CHF_2$, $CF_3$, alcoxyalkyle, aryloxyalkyle, cycloalkyle, alcényle (alkyle nonsubstitué), alkylène (cycloalkyle en $C_{3-7}$), -OCOR, aryle, hétéroaryle ou acétal, ou où deux groupes hydroxyles sont réunis sous la forme d'un cétal;
(f) un substituant halogéné tel que le fluor ou le chlore; (g) de l'hydrogène pour fournir un sucre déoxy,

où R, $R_1$ et $R_2$ sont indépendamment l'hydrogène ou un alkyle ou un aryle nonsubstitué ou substitué en $C_{1-10}$; ou un dérivé de ceux-ci.

2. Le composé de la revendication 1, dans lequel le résidu de sucre est un hexose ou un pentose, ou des formes substituées de ceux-ci.

3. Le composé de la revendication 2, dans lequel le résidu de sucre est un hexose sélectionné à partir du groupe se composant du glucose, du galactose ou du mannose.

4. Le composé de la revendication 2, dans lequel le résidu de sucre est une hexosamine.

5. Le composé de la revendication 4, dans lequel l'hexosamine est la galactosamine ou la glucosamine.

6. Le composé de l'une quelconque des revendications précédentes, dans lequel le résidu de sucre et le cyclitol sont reliés en α.

7. Le composé de l'une quelconque des revendications 1 à 5, dans lequel le résidu de sucre et le cyclitol sont reliés en β.

8. Le composé de l'une quelconque des revendications précédentes qui est sélectionné à partir du groupe se composant de :

*O*-(2-amino-2-désoxy-D-glucopyranosyl)-β(1,6)-D-3-*O*-méthyl-*chiro*-inositol;
*O*-(2-amino-2-désoxy-D-glucopyranosyl)-α(1,6)-D-3-*O*-méthyl-*chiro*-inositol;
*O*-(2-amino-2-désoxy-D-galactopyranosyl)α(1,6)-D-3-*O*-méthyl-*chiro*-inositol; et
1'-D-6-*O*-(2'-amino-2'-désoxy-β-D-galactopyranosyl)-3-*O*-méthyl-D-*chiro*-inositol;

ou un dérivé de ceux-ci.

9. Le composé de l'une quelconque des revendications précédentes, dans lequel le dérivé est un sel, un complexe de coordination avec un ion métallique, un ester, un acide ou une base libre, un hydrate ou un groupe protecteur.

**10.** Une composition comprenant un composé de l'une quelconque des revendications précédentes, en combinaison avec un support pharmaceutiquement acceptable.

**11.** Un composé de l'une quelconque des revendications 1 à 9 utilisable en thérapie.

**12.** Utilisation d'un composé de l'une quelconque des revendications 1 à 9 pour la préparation d'un médicament pour le traitement d'un état amélioré par l'administration d'un second messager d'inositol phosphoglycan (IPG) ou d'un antagoniste d'IPG.

# Scheme 1

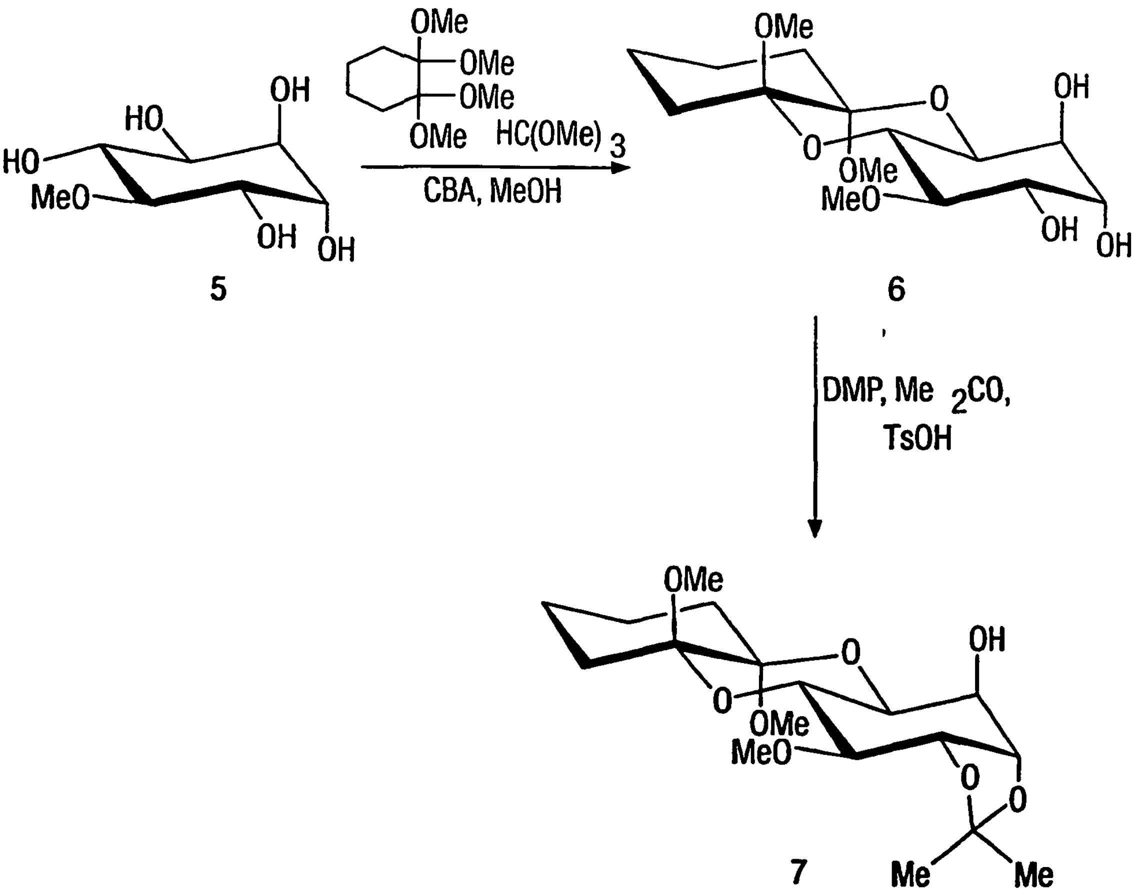


5

6

7

# Scheme 2

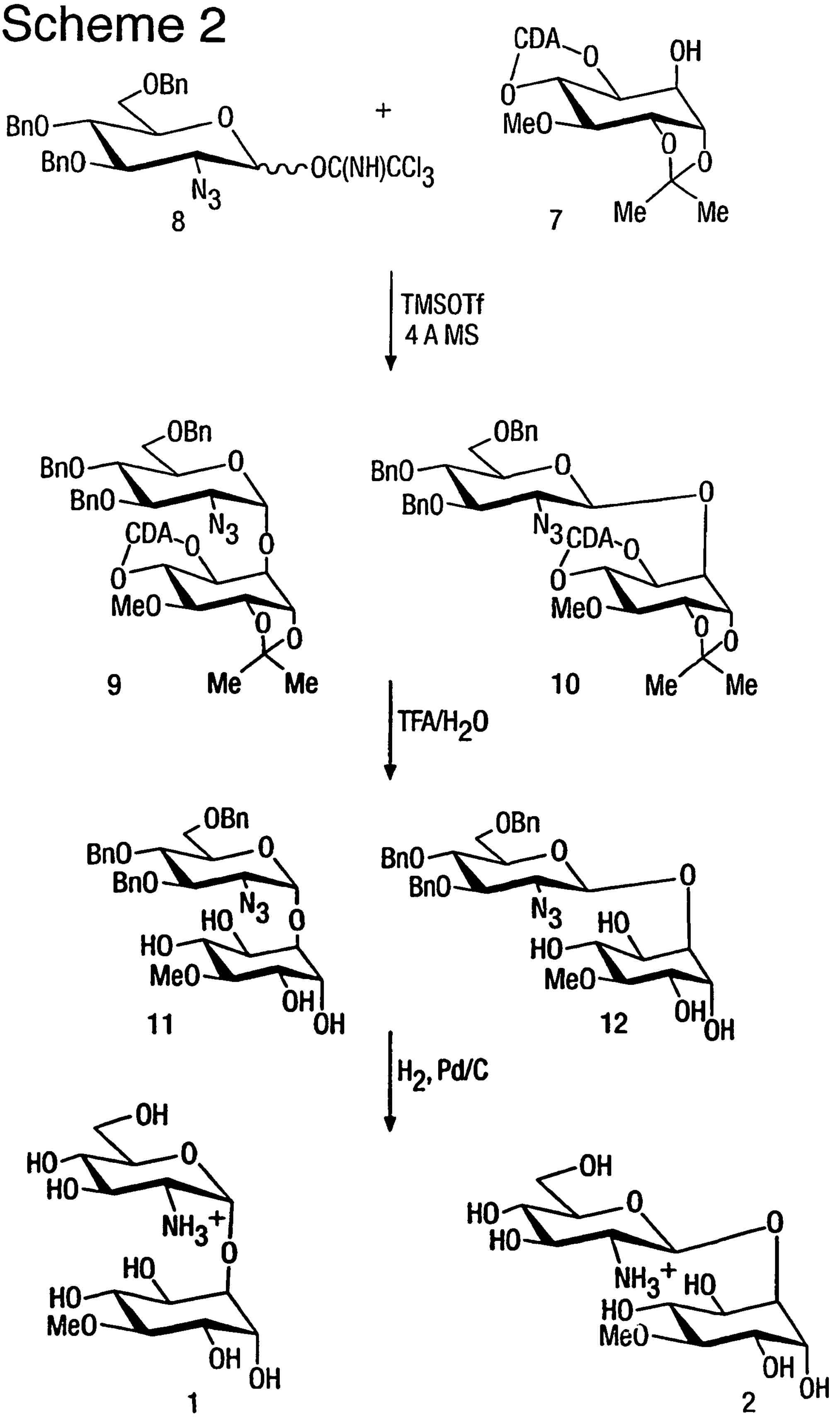


8 7 9 10 11 12 1 2

## Scheme 3

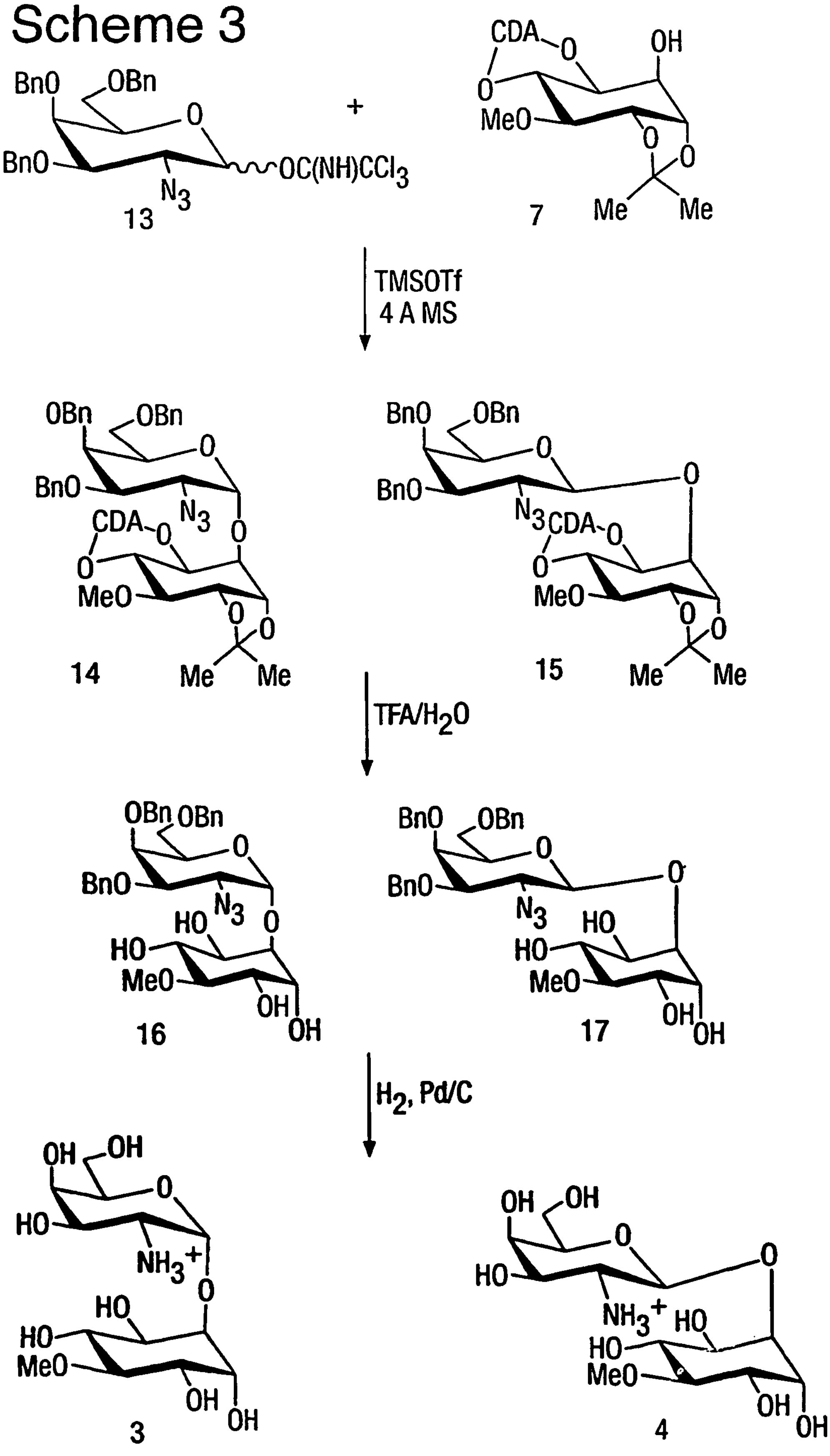

# Scheme 4

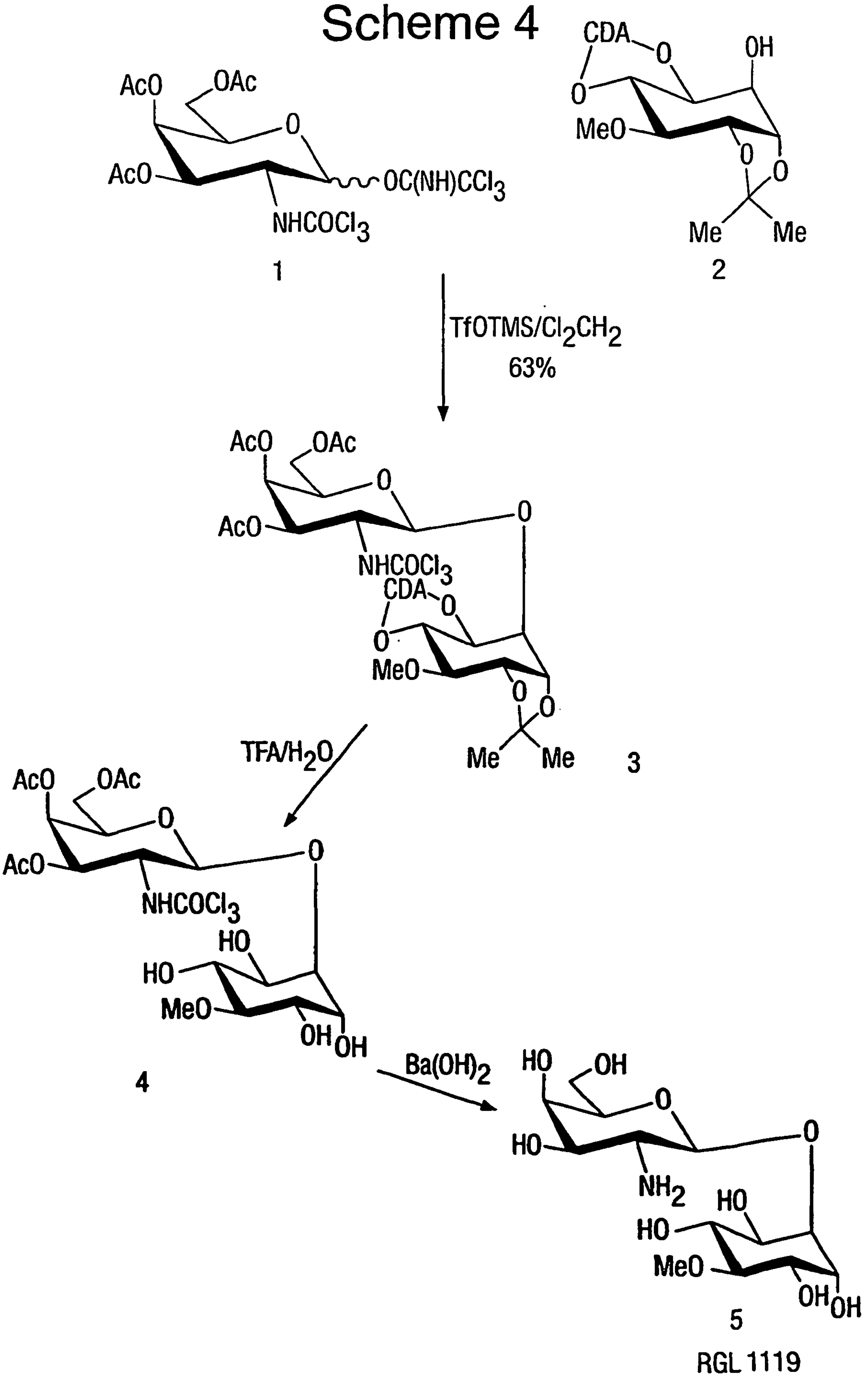


1

2

3

4

5

RGL 1119

Fig.1.

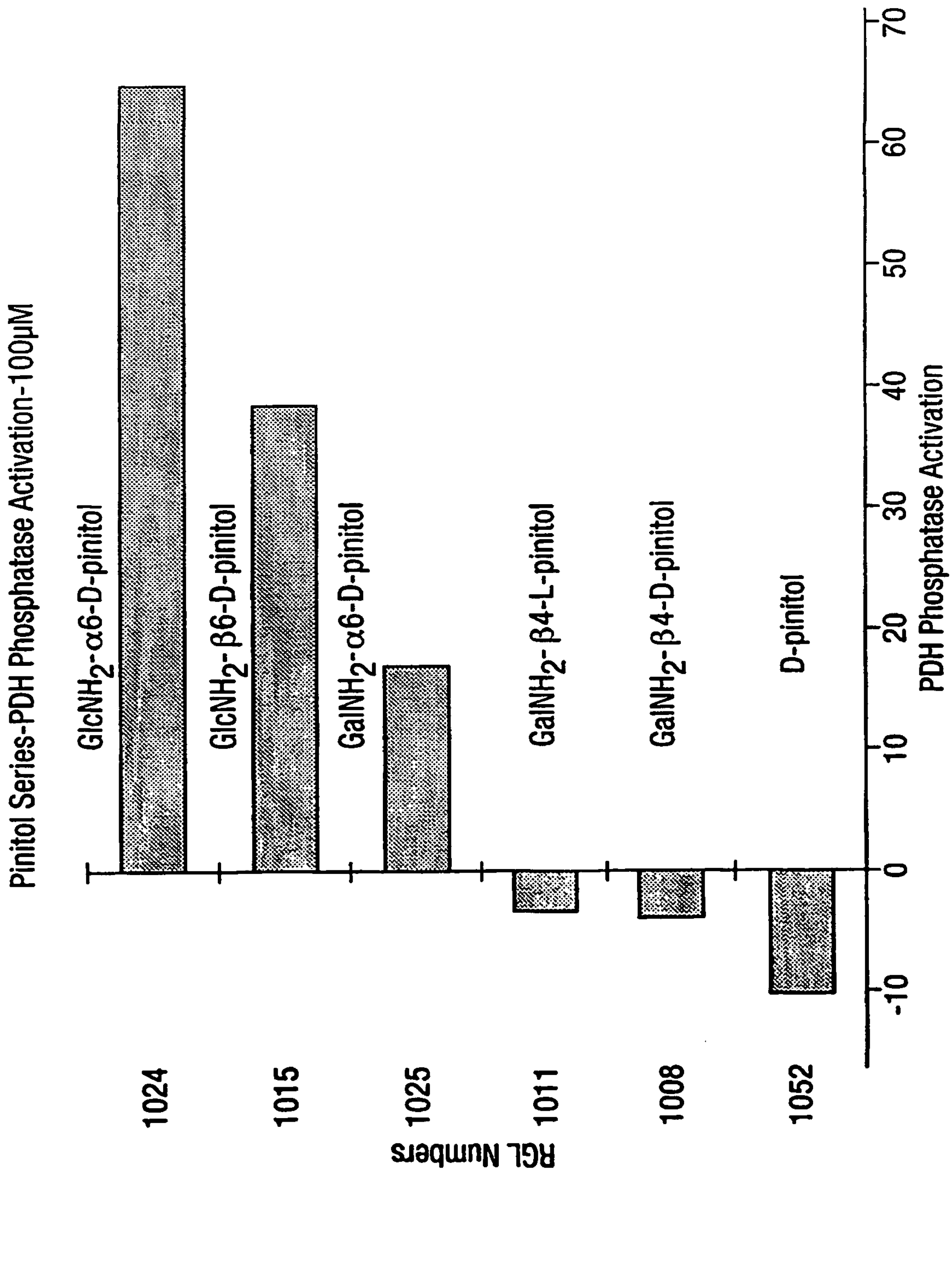
Pinitol Series-PDH Phosphatase Activation-100μM
RGL Numbers
1024
1015
1025
1011
1008
1052
GlcNH2-α6-D-pinitol
GlcNH2-β6-D-pinitol
GalNH2-α6-D-pinitol
GalNH2-β4-L-pinitol
GalNH2-β4-D-pinitol
D-pinitol
-10
0
10
20
30
40
50
60
70
PDH Phosphatase Activation